# FASCICULE DE BREVET EUROPEEN

(11) **EP 0 800 498 B1**
(45) Date de publication et mention de la délivrance du brevet: **07.11.2001**
(21) Numéro de dépôt: 95943280.8
(22) Date de dépôt: 27.12.1995
(51) Int. Cl.: C07C 15/08, C07C 7/00, C07C 7/12, C07C 7/14

(54) **PROCEDE DE SEPARATION DE PARAXYLENE COMPORTANT AU MOINS UN ETAGE DE CRISTALLISATION A HAUTE TEMPERATURE ET AU MOINS UN TRAITEMENT A LA TERRE SITUE EN AMONT DE LA ZONE D'ADSORPTION**
VERFAHREN ZUR ABTRENNUNG VON PARA-XYLOL DAS EINE KRISTALLISIERUNGSSTUFE AUF HOHER TEMPERATUR UND EINE, STROMAUFWARTS DER ADSORPTIONSSTUFE SITUIERTE, STUFE ZUR BEHANDLUNG MIT ERDE ENTHALT
METHOD FOR THE SEPARATION OF PARAXYLENE INVOLVING AT LEAST ONE HIGH TEMPERATURE CRYSTALLIZATION STAGE AND AT LEAST ONE CLAY TREATMENT LOCATED UPSTREAM OF THE ADSORPTION AREA

(30) Priorité: 29.12.1994 FR 9415896
(43) Date de publication de la demande: 15.10.1997
(73) Titulaire: INSTITUT FRANCAIS DU PETROLE, 92506 Rueil-Malmaison Cédex (FR)
(72) Inventeur: HOTIER, Gérard, F-92500 Rueil-Malmaison (FR); MIKITENKO, Paul, F-78590 Noisy-le-Roy (FR); MacPHERSON, Stuart, R., Piedmont, CA 94611 (US)
(74) Mandataire: Andreeff, François
(86) Numéro de dépôt international: FR9501738
(87) Numéro de publication internationale: WO9620907

(56) Documents cités:
- FR-A- 2 681 066

## Description

L'invention concerne un procédé de séparation et de préparation de paraxylène à partir d'un mélange d'hydrocarbures aromatiques comportant des isomères du xylène.

Les isomères du xylène sont l'orthoxylène, le métaxylène, le paraxylène et l'éthylbenzène. L'application principale de l'invention est l'obtention de paraxylène à un degré de pureté suffisant pour la synthèse par exemple de l'acide téréphtalique, utilisé dans la préparation de fibres synthétiques et notamment du polyester.

La demanderesse a décrit dans un brevet FR 2 681 066 (US 5 284 992) incorporé comme référence, un procédé de séparation et de récupération de paraxylène contenu dans une charge d'hydrocarbures comprenant essentiellement des hydrocarbures aromatiques à 8 atomes de carbone.

Ce procédé comprend la combinaison d'une étape d'enrichissement qui est une adsorption sélective sur un adsorbant en lit mobile simulé d'une charge contenant essentiellement les isomères du xylène qui permet d'enrichir substantiellement en paraxylène un premier effluent d'adsorption, et d'une purification qui est une cristallisation dans au moins une unité de cristallisation fonctionnant à haute température de l'effluent enrichi en paraxylène de façon à obtenir du paraxylène à très haute pureté, la liqueur mère étant recyclée à l'étape d'adsorption.

Cette cristallisation à haute température correspond à la seconde étape de cristallisation des procédés conventionnels de cristallisation (Chevron, Arco, qui comprennent en général une première cristallisation à basse température (-40 à -70°C) et une seconde cristallisation pour purifier à haute température (0 à -20°C par exemple) des cristaux obtenus préalablement refondus.

Par ailleurs, une seconde fraction appauvrie en paraxylène et donc enrichie en ortho et en méta-xylène ainsi qu'en éthylbenzène, délivrée par l'unité d'adsorption sélective, est envoyée dans une unité d'isomérisation pour augmenter sa concentration en paraxylène à une valeur proche de l'équilibre et sensiblement voisine ou supérieure à la composition de la charge initiale d'hydrocarbures et l'isomérat obtenu peut alors être recyclé à l'adsorption.

Dans l'enchaînement adsorption, cristallisation et isomérisation décrit, divers types d'impuretés peuvent apparaître dans les différents effluents et entraîner des perturbations dans la marche des unités qui nuisent au rendement obtenu et à la pureté du paraxylène récupéré.

Tout d'abord, lors de l'isomérisation de la fraction appauvrie en paraxylène, des hydrocarbures oléfiniques peuvent être produits en quantité variable suivant les valeurs des pressions partielles d'hydrogène introduit. La formation subséquente de polymères et leur passage dans l'unité d'adsorption peuvent entraîner de sérieux problèmes de circulation à travers l'adsorbant, voire sa désactivation.

De plus, des hydrocarbures paraffiniques et naphténiques à 8 et 9 atomes de carbone dont la volatilité est comprise entre celle du toluène, solvant de désorption par exemple, et celle des xylènes, sont des produits intermédiaires de la conversion de l'éthylbenzène en xylènes lors de l'isomérisation et leur accumulation peut s'avérer préjudiciable.

Par ailleurs, des hydrocarbures aromatiques à 9 atomes de carbone, présents en faible proportion et mal séparés dans des colonnes de distillation peuvent être nuisibles au procédé, tout comme des aldéhydes et des cétones plus lourds que la charge initiale, qui se forment lorsque de l'oxygène se trouve accidentellement dissout.

Enfin, un autre problème est lié à la présence de méthanol. Cet alcool est parfois rajouté en faible proportion dans les mélanges de xylènes à cristalliser pour éviter la cocristallisation d'eau et de paraxylène.

En effet, les mélanges de C₈ aromatiques secs sont relativement hygroscopiques et lors du passage dans les centrifugeuses de la suspension de cristaux de paraxylène dans la liqueur mère, de l'eau contenue dans l'air ambiant peut être absorbée dans la liqueur mère et cette eau peut éventuellement cristalliser en liaison avec la température de cette liqueur mère.

De plus, certains échangeurs peuvent présenter des fuites et de l'eau peut passer accidentellement dans le mélange à cristalliser.

L'objet de l'invention est de remédier à ces inconvénients et de limiter notamment la teneur de ces diverses impuretés dans la section adsorption pour chercher à l'optimiser, dans la mesure où l'adsorbant est très sensible aux impuretés de la charge de la zone d'adsorption. Un autre objet de l'invention est d'améliorer la récupération du paraxylène produit et de minimiser le coût énergétique, notamment de l'étape de purification.

La présente invention concerne un procédé de séparation et de récupération du paraxylène à partir d'une charge pouvant contenir du métaxylène, ce procédé comprenant en combinaison une opération d'adsorption et une opération de cristallisation. Selon l'invention au moins une partie de la charge et/ou des produits résultant de la cristallisation, par exemple la liqueur mère, subit une opération de traitement à la terre. La terre est choisie dans le groupe des silicoaluminates naturels activés ou non, la température est de 100 à 300 °C, la pression de 3 à 100 bar (1 bar = 10⁵Pa) et la vitesse horaire spatiale de 1 à 8. Au moins une partie de l'effluent résultant de l'opération de traitement à la terre est recyclée pour subir une opération d'adsorption.

La présente invention s'applique également à un procédé comportant en combinaison en plus des opérations d'adsorption et de cristallisation une étape d'isomérisation. Dans ce cas au moins une partie de l'isomérat pourra être traitée à la terre.

La présente invention concerne par ailleurs un procédé de séparation et de récupération du paraxylène à partir d'une charge pouvant contenir du métaxylène, ce procédé comprenant en combinaison une opération d'adsorption et une opération de cristallisation. Selon ce procédé, une partie au moins de la liqueur mère issue de l'opération de cristallisation est purifiée par distillation avant d'être recyclée à l'opération d'adsorption.

L'invention concerne également un procédé de séparation et de récupération de paraxylène contenu dans une charge d'hydrocarbures comprenant des hydrocarbures aromatiques en C₈, procédé dans lequel notamment :
- on met en contact dans au moins une zone d'adsorption, ladite charge contenant au moins du métaxylène, du paraxylène avec au moins un adsorbant en présence d'au moins un solvant de désorption approprié dans des conditions d'adsorption telles qu'on obtient une première fraction contenant du solvant, du métaxylène et éventuellement de l'éthylbenzène et/ou de l'orthoxylène et une seconde fraction contenant du solvant du paraxylène substantiellement enrichi ;
- on distille la première fraction en vue de récupérer un mélange métaxylène et éventuellement éthylbenzène, et/ou orthoxylène ;
- on isomérise au moins en partie ledit mélange dans des conditions appropriées dans au moins une zone d'isomérisation et l'on récupère un isomérat que l'on recycle au moins en partie vers la zone d'adsorption ;
- on distille ladite deuxième fraction en vue d'obtenir du paraxylène substantiellement enrichi et on procède à une cristallisation du paraxylène substantiellement enrichi avec obtention d'une part d'une liqueur mère que l'on recycle au moins en partie vers l'étape d'adsorption et d'autre part des cristaux de paraxylène.

Le procédé se caractérise en ce qu'on fait circuler au moins une partie choisie parmi au moins en partie la charge, au moins en partie l'isomérat et au moins en partie la liqueur mère dans au moins une zone de traitement à la terre ou matériau équivalent, ladite terre étant choisie dans le groupe des silicoaluminates naturels ou activés, la température étant de 100°C à 300 °C, la pression de 3 à 100 bar et la vitesse horaire spatiale de 1 à 8 et l'on récupère un premier effluent que l'on introduit au moins en partie dans la zone d'adsorption pour produire les dites première et deuxième fractions de l'étape soutirées de la zone d'adsorption.

On peut laver les cristaux de paraxylène avec un solvant de lavage approprié dans au moins une zone de lavage et l'on récupère des cristaux de paraxylène à un très grand degré de pureté.

Par cristallisation à haute température du paraxylène, on entend une cristallisation d'une solution ou suspension de paraxylène déjà enrichie en paraxylène qui correspond à ce que la littérature appelle une étape de purification. Par exemple, le brevet US 2 866 833 incorporé comme référence, mentionne une étape de purification du paraxylène pouvant aller jusqu'à -34°C.

Une partie au moins de la charge d'hydrocarbures peut être prétraitée dans un réacteur de traitement à la terre. Il peut être avantageux que l'isomérat soit envoyé dans un réacteur de traitement à la terre avant d'être envoyé dans l'adsorption. Ces réacteurs peuvent être indépendants ou ne former qu'un réacteur unique, éventuellement commun à celui qui traite la liqueur mère.

Ces traitements à la terre permettent d'éliminer au moins en partie les oléfines créées notamment dans l'étape d'isomérisation et une partie au moins des impuretés lourdes, qui circulent dans la boucle adsorption, cristallisation et isomérisation.

Différentes variantes peuvent être envisagées :

La liqueur mère peut être avantageusement introduite au moins en partie dans une colonne de distillation en aval de la zone d'isomérisation. Cette colonne traite aussi l'effluent de la zone d'isomérisation et délivre une fraction de tête contenant des composés légers (eau, méthanol, C₇⁻) et une autre fraction contenant un mélange distillé de liqueur mère et d'isomérat que l'on introduit ensuite dans la zone de traitement à la terre.

Une fraction de queue de distillation contenant des composés lourds peut aussi être soutirée de cette colonne de distillation, ce qui permet de diminuer la taille des installations aval.

Une partie de la liqueur mère peut aussi être mélangée à l'effluent quel qu'il soit, sortant de la zone de traitement à la terre, que ce soit l'effluent résultant de la circulation de l'isomérat, de la liqueur mère et de la charge, dans la zone de traitement à la terre ou l'effluent résultant de la circulation dans la zone de traitement à la terre, de ces derniers et de la fraction de distillation contenant ledit mélange distillé de liqueur mère et d'isomérat, avant d'être introduits dans la zone d'adsorption sélective.

L'effluent résultant de ces dernières variantes peut être distillé dans au moins une colonne à distiller (colonne dite rerun) qui délivre une fraction de queue contenant des composés lourds et une fraction de tête qui est introduite dans la zone d'adsorption avec éventuellement une partie de la liqueur mère.

Les conditions d'adsorption ou d'élimination des composés indésirables sur la terre sont, en règle générale, les suivantes :
- Température : 100 à 300° C, de préférence 160 à 230° C
- Vitesse spatiale horaire : 1 à 8, de préférence 1 à 4 (Volume horaire de charge par volume de terre)
- Type de terre : aluminosilicates naturels activés ou non, par exemple la terre référencée F54 chez ENGELHARD.
- Pression : 3 à 100 bar, de préférence 4 à 20 bar (1 bar = 10⁵Pa).

La colonne de distillation suivant l'isomérisation a en général les caractéristiques suivantes :
- Pression : 1 à 20 bar, de préférence 3 à 8 bar
- Température en fond : 150 à 280° C, de préférence 200 à 240° C
- Nombre de plateaux : 30 à 80, de préférence 50 à 70.

La colonne à distiller dite rerun située entre la zone de traitement à la terre et la zone d'adsorption sélective a habituellement les caractéristiques suivantes :
- Pression : 1 à 20 bar, de préférence 3 à 8 bar
- Température en fond : 160 à 290° C, de préférence 210 à 250° C
- Nombre de plateaux : 40 à 200, le plus souvent 50 à 90.

Selon une autre caractéristique de l'invention, on peut être amené à contenir la teneur en constituants de volatilité intermédiaire à un niveau tolérable. Dans ce cas, au moins une partie de la liqueur mère peut être purgée avant d'être introduite dans la zone de traitement à la terre.

Il peut aussi être avantageux de purger au moins en partie le solvant de désorption résultant des étapes (b) et (d) de distillation de la fraction appauvrie ou de la fraction enrichie en paraxylène avant qu'il ne soit recyclé et de compenser la purge du solvant par un apport de solvant frais, soit dans la charge soit en amont de la zone d'adsorption.

Comme il a été indiqué, il est possible de recycler la liqueur mère de cristallisation en des endroits différents de l'installation selon l'importance des teneurs en composés indésirables mais il peut être avantageux de combiner entre eux ces différents recyclages, par exemple, lorsqu'il s'agit de réutiliser des équipements existants pour la distillation de l'isomérat, le traitement à la terre ou la distillation dite de rerun et lorsque l'un de ces équipements est déjà opéré à son débit maximum.

On peut aussi combiner ces différents recyclages et ces purges lorsqu'on cherche à faire baisser la teneur en une impureté dans la boucle sans chercher à l'éliminer totalement.

L'étape de cristallisation de la fraction enrichie en paraxylène est en général réalisée, comme il a été mentionné ci-dessus, à haute température par exemple entre + 10 et-30°C et de préférence + 10 et -25°C. La température est habituellement choisie en fonction de la concentration en paraxylène que l'on souhaite avoir dans la liqueur mère, et du coût économique de l'opération de cristallisation.

On peut dans ces conditions réaliser une cristallisation du paraxylène en un seul étage lorsque le paraxylène recueilli par exemple en tant qu'extrait de l'unité d'adsorption a une pureté supérieure, par exemple, à 85%.

Mais il peut être avantageux, notamment pour des raisons économiques de réaliser une cristallisation en plusieurs étages, par exemple une première cristallisation à une température T1 comprise entre +10 et -10°C, et une seconde cristallisation à une température T2, inférieure à T1, comprise entre 0 et -25°C.

Selon un premier mode de réalisation de l'invention, on effectue une cristallisation à un seul étage, du paraxylène de l'étape (d), on récupère une suspension de cristaux dans la liqueur mère, on sépare les cristaux de la liqueur mère dans une zone de séparation, on lave les cristaux obtenus avec le solvant de lavage, on récupère une liqueur de lavage, on recycle une partie au moins de la liqueur mère et une partie au moins de la liqueur de lavage et on fond complètement les cristaux de paraxylène de façon à obtenir un courant liquide de paraxylène fondu.
Plusieurs modes de réalisations de cette première variante sont décrits conformément aux revendications 17, 18, 19 et 20.

Selon un second mode de réalisation de l'invention mettant en oeuvre une première variante d'une cristallisation à deux étages de la fraction enrichie en paraxylène, on effectue une première cristallisation du paraxylène de l'étape (d) dans une première zone de cristallisation à une température T₁, on récupère une suspension de premiers cristaux de paraxylène dans une première liqueur mère, on sépare les premiers cristaux de la première liqueur mère dans une première zone de séparation, on recycle une partie au moins de la première liqueur mère vers la zone d'adsorption, on fond les premiers cristaux séparés et on les cristallise dans une deuxième zone de cristallisation à une température T2 supérieure à T1, on récupère une suspension de seconds cristaux de paraxylène dans une seconde liqueur mère, on sépare les seconds cristaux de la seconde liqueur mère dans une seconde zone de séparation, on lave les seconds cristaux avec le solvant de lavage approprié dans la seconde zone de séparation, on récupère une liqueur de lavage, on recycle au moins en partie la seconde liqueur mère et une partie au moins de la liqueur de lavage vers la première zone de cristallisation et éventuellement en partie vers la seconde zone de cristallisation et on fond complètement les seconds cristaux de paraxylène de façon à obtenir un courant liquide de paraxylène fondu.

Par exemple, la température T1 de la première cristallisation peut être de -5 à -25°C alors que la température T2 de la seconde cristallisation peut être de +10 à -5°C.

Plusieurs modes de réalisation relatifs à cette température (T2) de deuxième zone de cristallisation supérieure à celle (T1) de la première zone de cristallisation sont décrits conformément aux revendications 22, 23 et 24.

Selon un autre mode de réalisation de l'invention, mettant en oeuvre une deuxième variante d'une cristallisation à deux étages, on effectue une première cristallisation du paraxylène de l'étape (d) dans une première zone de cristallisation à une température T1, on récupère une suspension de premiers cristaux de paraxylène dans une première liqueur mère, on sépare les premiers cristaux de la première liqueur mère dans une première zone de séparation, on lave éventuellement les premiers cristaux avec le solvant de lavage dans la première zone de séparation, on cristallise une partie au moins de la première liqueur mère dans une seconde zone de cristallisation à une température T2 inférieure à la température T1, on récupère une suspension de seconds cristaux de paraxylène dans une seconde liqueur mère, on sépare les seconds cristaux de la seconde liqueur mère dans une seconde zone de séparation, on lave les seconds cristaux avec le solvant de lavage dans la seconde zone de séparation, on récupère une seconde liqueur mère que l'on recycle au moins en partie vers la zone d'adsorption, on mélange les premiers et les seconds cristaux de paraxylène, on les fond complètement et on récupère un courant de paraxylène fondu.

Par exemple, la première cristallisation peut être réalisée à une température T1 comprise entre +10 et -10°C alors que la seconde T2, inférieure à T1, peut être réalisée entre 0 et -25°C.

D'autres caractéristiques de ce mode de réalisation sont décrits conformément aux revendications 26 et 27.

Selon un autre mode de mise en oeuvre de l'invention comportant une cristallisation à plusieurs étages, on effectue une première cristallisation du paraxylène de l'étage (d) et de cristaux partiellement fondus ou éventuellement fondus, recyclés provenant d'une seconde cristallisation ci-après, dans une première zone de cristallisation à une température T1, on récupère une suspension de cristaux de paraxylène dans une première liqueur mère, on sépare les cristaux de la première liqueur mère dans une première zone de séparation, on lave lesdits cristaux, on les fond et on récupère un courant de paraxylène fondu, on cristallise un partie au moins de la première liqueur mère dans une seconde zone de cristallisation à une température T2 inférieure à la température T1, on récupère une suspension de seconds cristaux de paraxylène dans une seconde liqueur mère, on sépare les seconds cristaux de la seconde liqueur mère dans une seconde zone de séparation, on récupère une seconde liqueur mère que l'on recycle au moins une partie vers la zone d'adsorption, on fond éventuellement les seconds cristaux et on recycle les cristaux éventuellement fondus vers la première zone de cristallisation pour les recristalliser avec le paraxylène de l'étape (d) à la température T1.

Une autre variante peut consister à fondre partiellement les seconds cristaux et à recycler la suspension obtenue vers la première zone de cristallisation pour la recristalliser avec le paraxylène de l'étape (d) à la température T1.
Selon un autre mode de mise en oeuvre particulièrement avantageux, on effectue une première cristallisation du paraxylène de l'étape (d) dans une première zone de cristallisation à une température T1, on récupère une suspension de premiers cristaux de paraxylène dans une première liqueur mère, on sépare les premiers cristaux de la première liqueur mère dans une première zone de séparation, on cristallise une partie au moins de la première liqueur mère dans une seconde zone de cristallisation à une température T2 inférieure à T1, on sépare les seconds cristaux de la seconde liqueur mère dans une seconde zone de séparation, on récupère la seconde liqueur mère que l'on recycle au moins une partie vers la zone d'adsorption, on récupère les premiers cristaux et les seconds cristaux et on les lave dans au moins une zone de séparation et de lavage à contre courant avec le solvant de lavage approprié, on récupère d'une part une liqueur de lavage qui est recyclée au moins en partie vers la première zone de cristallisation, on récupère d'autre part des cristaux de paraxylène, on les fond complètement dans une zone de fusion et on recueille un courant de paraxylène fondu.

Bien entendu on ne sortira pas du cadre de la présente invention en utilisant un autre solvant de désorption que le toluène tel notamment le paradiéthylbenzène (PDEB), ce solvant étant plus lourd que les xylènes, sera récupéré en fond de certaines colonnes alors que le toluène l'était en tête.

L'invention sera mieux comprise au vu des figures suivantes illustrant de manière non limitative plusieurs modes de mise en oeuvre de l'invention, parmi lesquelles :
- la figure 1 représente de manière schématique le procédé et les diverses possibilités de recyclage de l'isomérat et de la liqueur mère de cristallisation vers l'adsorption via un traitement à la terre.
- les figures 2 et 3 illustrent une purification du paraxylène par cristallisation à un seul étage.
- les figures 4 et 5 montrent une purification du paraxylène par cristallisation à double étage, le premier étant plus froid que le second.
- les figures 6 à 10 représentent une purification du paraxylène par cristallisation à double étage, le premier étant plus chaud que le second.

Les conditions opératoires et l'adsorption en lit mobile simulé (contre courant par exemple) sont choisies de façon que la première fraction contenant le métaxylène, l'orthoxylène et l'éthylbenzène soit un raffinat et la deuxième fraction contenant essentiellement le paraxylène soit un extrait. Ces conditions sont décrites dans le brevet US 5 284 992.

On véhicule par une ligne 1 une charge comprenant environ 20% d'éthylbenzène, 18% de paraxylène, 45% de métaxylène et 17% d'orthoxylène. On y joint par une ligne 2 un effluent recyclé dont la teneur en éthylbenzène est sensiblement plus faible, typiquement 8 à 13% et qui contient des impuretés. Par des lignes 3 et 30, on introduit un autre effluent recyclé dont la teneur en paraxylène est plus forte, typiquement 25 à 45%. Une ligne 4 récupère la charge et ces deux effluents, elle véhicule un mélange de composition approximative, paraxylène 20 à 22,5, éthylbenzène 9 à 14%, orthoxylène 20 à 22,5%, métaxylène 45 à 50% qui est introduit dans une zone d'adsorption 8 en contre-courant simulé comprenant une ou plusieurs colonnes 6 et/ou 7 remplies d'un adsorbant zéolitique, chacune des colonnes étant divisée en un nombre limité de lits, le nombre des lits de chaque colonne pouvant être compris entre 4 et 20, la productivité exprimée par rapport au paraxylène produit étant d'environ 0,07 m³ par m³ de tamis et par heure exprimée aux conditions ambiantes. On désorbe par du toluène, à raison d'environ 1,45 m³ de toluène par m³ de charge, la température opératoire étant à peu près 160°C. On soutire de cette unité par une ligne 10 un raffinat appauvri en paraxylène contenant essentiellement du toluène, du métaxylène, de l'éthylbenzène et de l'orthoxylène et par une ligne 9 un extrait de composition enrichie en paraxylène contenant essentiellement du toluène et du paraxylène, l'impureté majeure étant l'éthylbenzène. Le raffinat est introduit dans une colonne à distiller 12 (température de tête 125°C, température de fond 160°C par exemple). On soutire en tête par une ligne 14 du toluène (environ 30 % de la quantité introduite dans l'adsorption par exemple) contenant par exemple, moins de 2 % de composés C₈ aromatiques et l'on soutire en fond de cette colonne par une ligne 15 un liquide (raffinat débarrassé du solvant) riche en éthylbenzène, en métaxylène et en orthoxylène et appauvri en paraxylène (moins de 3 % par exemple) que l'on envoie dans une unité d'isomérisation 21. Ce raffinat est mis en contact avec de l'hydrogène introduit par une ligne 20 et avec un catalyseur à base de mordénite et de platine sur alumine à environ 380°C. Une ligne 22 conduit l'isomérat de la sortie du réacteur vers un ballon de séparation des constituants gazeux (non représenté sur la figure), puis vers une colonne à distiller 23 (température de tête 90°C, température de fond 160°C par exemple). On soutire en tête par une ligne 24 des hydrocarbures de C₁ à C₅, de l'hexane, du cyclohexane, du benzène et du toluène et en fond de cette colonne par une ligne 2, un effluent contenant 8 à 13% d'éthylbenzène, 21 à 24% de paraxylène, 21 à 24% d'orthoxylène, de 45 à 50% de métaxylène et des impuretés, qui est recyclé vers la zone d'adsorption 8.

La ligne 9 introduit l'extrait dans une colonne à distiller 16 d'où l'on soutire en tête du toluène à moins de 2 % de composés C₈ aromatiques (environ 70 % de la quantité introduite dans l'adsorption par exemple) qui est recyclé par les lignes 17 et 11 vers l'alimentation en solvant de désorption de l'unité d'adsorption. En fond de colonne 16 à environ 160°C on soutire un courant enrichi en paraxylène (à environ 90% de paraxylène) au moyen d'une ligne 19 qui le conduit dans une unité de cristallisation 5a à un étage par exemple fonctionnant à environ -10°C. Dans cette unité 5a, 5b on produit des cristaux de paraxylène qui sont en suspension dans une liqueur mère. Les cristaux sont séparés dans au moins une centrifugeuse 5b par exemple et lavés dans la centrifugeuse. On recueille d'une part une liqueur mère appauvrie en paraxylène (environ 54 %) qui est recyclée par la ligne 3 vers la zone d'adsorption 8 via des zones de traitement à la terre et de distillation discutées plus loin et d'autre part des cristaux de paraxylène, que l'on fond. Le solvant de lavage, le toluène par exemple est amené par une ligne 18 et peut provenir comme représenté sur la figure, de l'unité de distillation du raffinat 12 et/ou encore de l'unité de distillation de l'extrait 16. On récupère de l'unité 5b, et après une distillation des cristaux fondus non représentée, du paraxylène liquide de pureté par exemple égale à 99,75% par une ligne 25 et du toluène que l'on recycle (ligne non représentée).

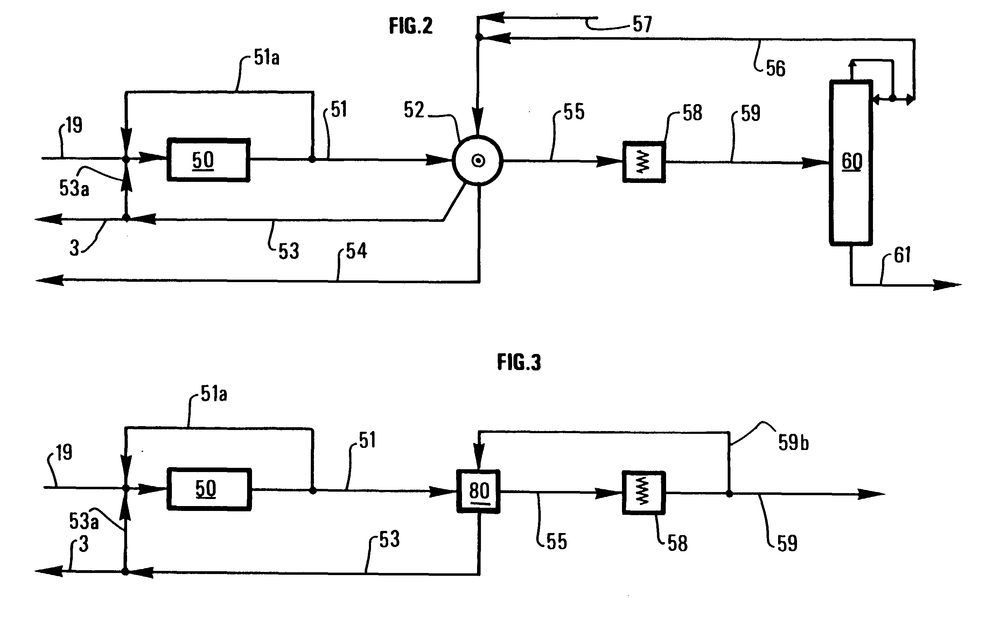

La figure 2 illustre plus précisément les étapes de cristallisation et de traitement aval des cristaux de paraxylène avec un lavage au toluène. Selon cette figure, l'unité de cristallisation 50 à un seul étage reçoit la charge de cristallisation, (l'extrait distillé), par la ligne 19. On récupère par une ligne 51 des cristaux en suspension dans une liqueur mère qui sont séparés au moins en partie dans au moins une centrifugeuse 52. Une liqueur mère contenant par exemple 54 % de paraxylène est soutirée et recyclée au moins en partie vers la zone d'adsorption 8 par les lignes 53 et 3, une autre partie pouvant être recyclée vers la zone de cristallisation 50 par une ligne 53a. Une partie des cristaux en suspension dans la liqueur mère peut aussi être recyclée dans la zone de cristallisation par une ligne 51a.

On introduit ensuite, comme solvant de lavage, dans la centrifugeuse 52, du toluène recyclé provenant d'une colonne de distillation 60 amené par une ligne 56 et du toluène apporté par une ligne 57 de solvant frais provenant de la ligne 18. Une liqueur de lavage est recueillie séparément par une ligne 54 raccordée à la centrifugeuse 52, et elle est recyclée au moins en partie dans l'unité de distillation du raffinat 12.

Les cristaux lavés de paraxylène sont soutirés par une ligne 55, fondus complètement dans une zone de fusion 58 et introduits dans la colonne de distillation 60 par une ligne 59. On récupère en fond de colonne du paraxylène liquide de très haute pureté et en tête de colonne, du toluène qui est recyclé en partie au moins dans la centrifugeuse 52. On a utilisé au moins une centrifugeuse pour séparer les cristaux et la liqueur mère. On aurait pu utiliser aussi au moins un filtre rotatif à la place de la centrifugeuse.

Selon une variante non illustrée, la centrifugeuse peut être remplacée par au moins une colonne de séparation et de lavage à contre-courant, décrite dans les brevets US 4 475 355, US 4 481 169, incorporés comme références, la colonne NIRO par exemple. Dans ce cas, la liqueur mère et la liqueur de lavage sont une seule et même liqueur qui est éventuellement distillée avant d'être recyclée, en partie au moins, vers la zone d'adsorption, via les zones de traitement à la terre et de distillation, et en partie éventuellement dans la zone de cristallisation.

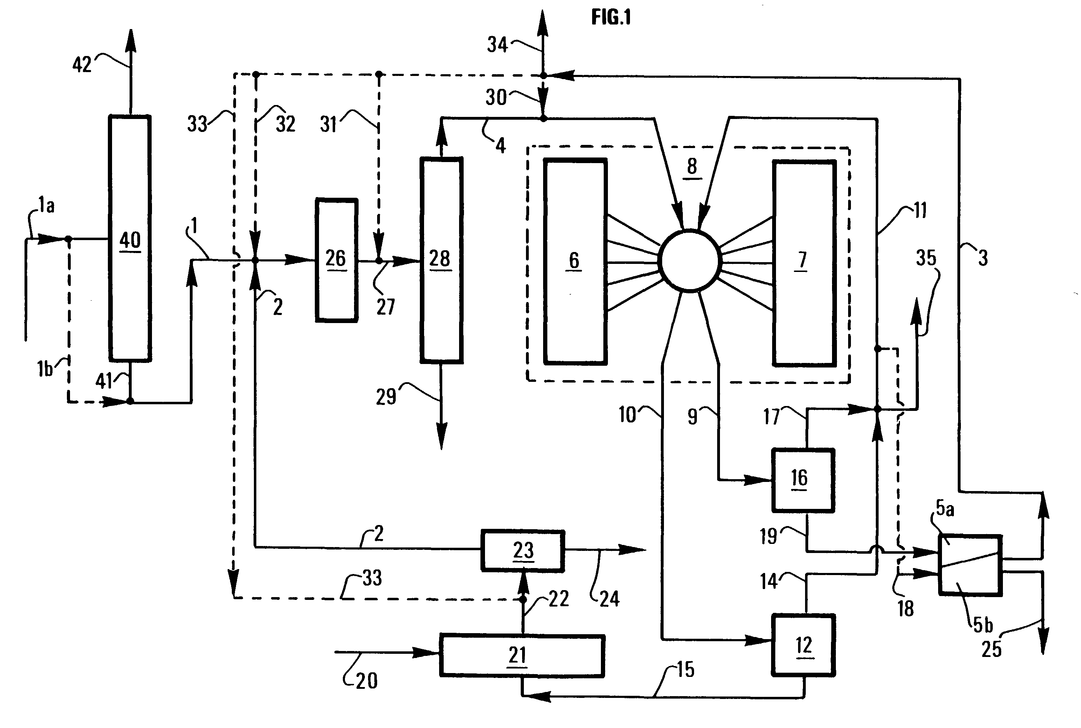

Selon une autre variante illustrée par la figure 3, on peut utiliser un autre solvant de lavage tel que le paraxylène fondu provenant de la zone de fusion 58. Dans ce cas de figure comportant les mêmes références pour les mêmes organes que ceux de la figure 1, une partie au moins du paraxylène fondu est introduite à contre-courant de la suspension 51, par une ligne 59b dans une colonne à contre-courant 80, type colonne NIRO par exemple et est utilisée pour laver dans la colonne les cristaux de paraxylène. Une partie au moins du paraxylène fondu introduit dans la colonne y cristallise.

Les cristaux récupérés de la colonne par la ligne 55 sont ensuite fondus dans la zone de fusion 58 et le paraxylène liquide de très haute pureté est récupéré par la ligne 59.

La liqueur de lavage et la liqueur mère sont récupérées en même temps par la ligne 53 et sont recyclées vers la zone d'adsorption 8, une partie pouvant être recyclée vers la zone de cristallisation 50.

Si l'on utilisait un solvant de lavage, par exemple le pentane, autre que le solvant de désorption (toluène) et le paraxylène fondu, on reproduirait le procédé décrit selon la figure 2, sauf que la liqueur de lavage provenant de la centrifugeuse 52 devrait être débarrassée du solvant par une distillation subséquente (non représentée sur la figure) avant d'être recyclée à l'adsorption ou à la cristallisation. Le solvant distillé est alors recyclé dans la centrifugeuse.

On pourrait également reproduire le procédé selon la figure 3 avec une colonne de lavage et à contre courant et un solvant de lavage autre que le paraxylène fondu et que le solvant de désorption. Dans ce cas, après la fusion complète des cristaux, on distille un courant de paraxylène fondu contenant du solvant de lavage, on récupère, en tête du solvant que l'on recycle au moins en partie dans la colonne de lavage et en fond, du paraxylène de très haute pureté. La liqueur mère comprenant la liqueur de lavage est soutirée de la colonne de lavage, distillée et recyclée en partie au moins vers la zone d'adsorption sélective et éventuellement en partie vers la zone de cristallisation.

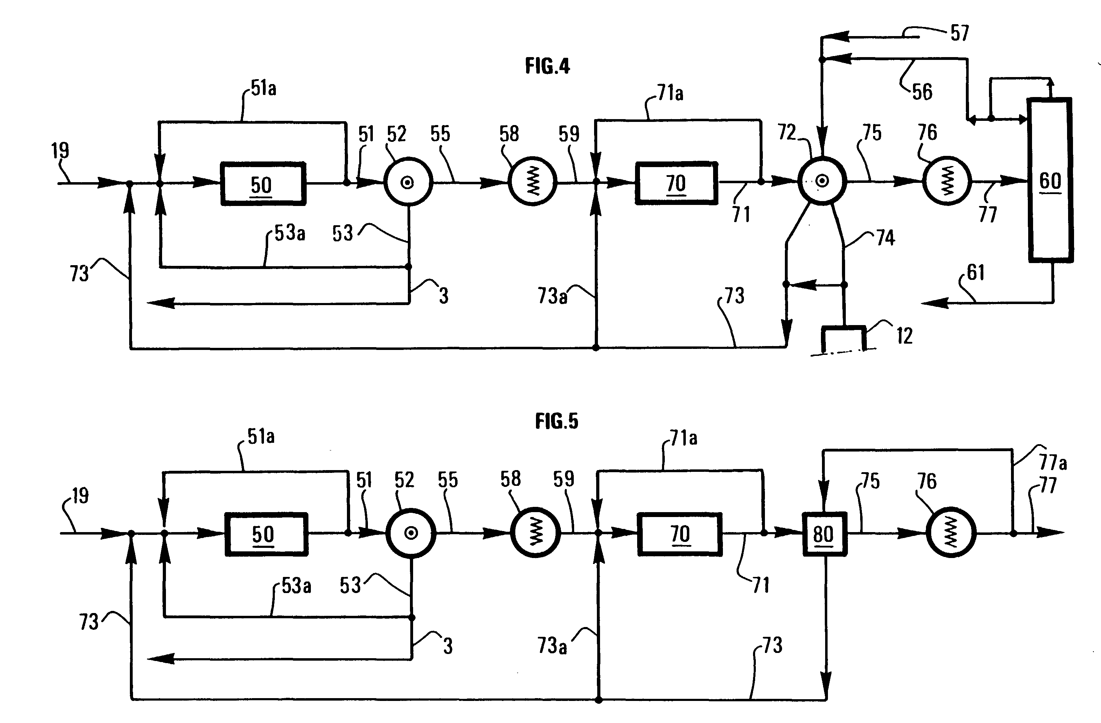

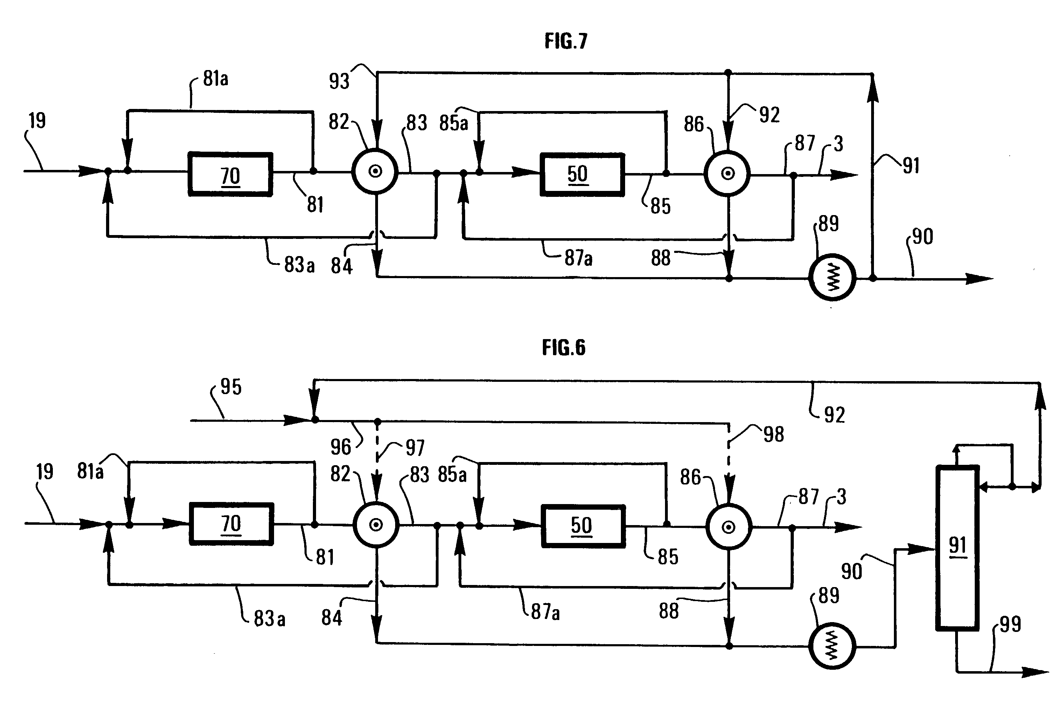

Les figures 4 et 5 illustrent une cristallisation à deux étages, la température du second étage de cristallisation étant plus élevée que celle du premier étage. Selon la figure 4, la première unité de cristallisation 50, à -20°C par exemple, reçoit la charge de cristallisation, (l'extrait distillé de l'adsorption), par la ligne 19. Sa pureté est d'environ 80%. On récupère par une ligne 51 des cristaux en suspension dans une liqueur mère qui sont séparés dans une première centrifugeuse 52. Une première liqueur mère contenant par exemple 40% de paraxylène est soutirée et recyclée au moins en partie vers la zone d'adsorption 8 par les lignes 53 et 3 via les zones de traitement à la terre et de distillation, l'autre partie pouvant être recyclée vers la première cristallisation.

Les cristaux récupérés par une ligne 55 sont fondus dans une zone de fusion 58 et introduits par une ligne 59 dans une seconde unité 70 de cristallisation opérant par exemple à 0°C. On récupère une suspension de seconds cristaux par une ligne 71 que l'on introduit dans au moins une seconde centrifugeuse 72 ou un filtre rotatif.

On récupère une seconde liqueur mère par une ligne 73, que l'on recycle au moins en partie vers la première unité de cristallisation 50 et éventuellement en partie vers la seconde zone de cristallisation. On lave les cristaux séparés avec le solvant de désorption (toluène par exemple) utilisé comme solvant de lavage, que l'on introduit dans la centrifugeuse par une ligne 56 et un appoint 57 provenant de la ligne 17 notamment et on soutire une liqueur de lavage 74 que l'on recycle au moins en partie vers la première unité de cristallisation 50 et/ou vers la seconde unité de cristallisation 70 après l'avoir éventuellement distillée. On aurait pu aussi la recycler vers l'unité de distillation du raffinat (étape b).

On récupère , par ailleurs, à partir d'une ligne 75 reliée à la centrifugeuse 72 les seconds cristaux que l'on fond complètement dans une zone de fusion 76 et on recueille par la ligne 77 du paraxylène fondu que l'on distille dans la colonne de distillation 60. Le toluène récupéré en tête est recyclé par la ligne 56 tandis que le paraxylène de très haute pureté est soutiré en fond de colonne par la ligne 61.

La centrifugeuse 72 aurait pu être remplacée par une colonne de lavage à contre courant. Dans ce cas, la seconde liqueur mère contenant du toluène de lavage peut être distillée comme c'est le cas avec un seul étage avant d'être recyclée, et le toluène de lavage renvoyé vers la colonne de lavage.

La figure 5, illustre l'utilisation d'une colonne à contre-courant type colonne NIRO comme seconde zone de séparation et de lavage des seconds cristaux à la place de la centrifugeuse 72 de la figure 4 ou d'un filtre rotatif, et qui utilise comme solvant de lavage, non pas du toluène mais une partie du courant de paraxylène fondu. Selon cette figure 5, comprenant les mêmes organes que ceux de la figure 4, on récupère les seconds cristaux en suspension dans la seconde liqueur mère par une ligne 71 en provenance de la seconde unité de cristallisation et on l'introduit dans la colonne NIRO 80 qui est alimentée en solvant de lavage par une partie du paraxylène fondu recueilli par une ligne 77a. On recueille par une ligne 75 des cristaux de paraxylène de très haute pureté que l'on fond dans la zone de fusion 76 et on récupère un courant de paraxylène fondu par la ligne 77. La seconde liqueur mère à 70 % par exemple de paraxylène et la liqueur de lavage sont récupérées en même temps et sont recyclées au moins en partie vers la première unité de cristallisation 50 par une ligne 73 et éventuellement en partie vers la seconde unité de cristallisation.

Selon une autre variante non illustrée par les figures, le solvant de lavage peut être un solvant autre que le courant de paraxylène fondu et le solvant de désorption, par exemple du pentane. Dans ce cas, on distille le courant de paraxylène fondu, de façon à récupérer en tête du solvant de lavage que l'on recycle au moins en partie dans la seconde zone de séparation et en fond, du paraxylène de très haute pureté, on distille la liqueur mère comprenant la liqueur de lavage avant de la recycler vers la première zone de cristallisation et éventuellement en partie vers la seconde zone de cristallisation si la seconde zone de séparation est une colonne de lavage à contre courant (colonne NIRO par exemple).

En revanche, si la seconde zone de séparation est une centrifugeuse ou un filtre rotatif, on recycle la liqueur mère vers la première zone de cristallisation et éventuellement en partie vers la seconde zone de cristallisation et on distille la liqueur de lavage avant de la recycler vers la première zone de cristallisation et éventuellement en partie vers la seconde zone de cristallisation.

Un recyclage des premiers cristaux en suspension 51a et des seconds cristaux en suspension 71a peut être envisagé, respectivement dans la première et dans la seconde zone de cristallisation.

Les figures 6 et 7 illustrent une cristallisation du paraxylène à deux étages, la température du second étage de cristallisation de la liqueur mère étant plus basse que celle du premier étage.

Selon la figure 6, la charge de cristallisation (l'extrait distillé de l'adsorption) est introduite par la ligne 19 dans la première unité de cristallisation 70 qui fonctionne vers 0°C. On récupère par une ligne 81 des premiers cristaux en suspension dans une première liqueur mère, qui sont séparés dans au moins une première centrifugeuse 82, lavés par du toluène amené par une ligne 97 et récupérés par une ligne 84. La première liqueur mère contenant par exemple 70 % de paraxylène est introduite au moins en partie, par une ligne 83 dans la seconde unité de cristallisation 50 opérant à -10° C. Une autre partie, peut être recyclée dans la première unité de cristallisation par une ligne 83a. On récupère par une ligne 85 des seconds cristaux en suspension dans une seconde liqueur mère, et on les sépare dans au moins une seconde centrifugeuse 86. On les recueille par une ligne 88 après les avoir lavés par du toluène que' l'on a introduit par une ligne 98 dans la seconde centrifugeuse . La seconde liqueur mère recueillie par la ligne 87 contient une fraction du toluène de lavage ; elle est recyclée au moins en partie vers la zone d'adsorption 8 via les zones de traitement à la terre et de distillation et éventuellement en partie par une ligne 87a vers la seconde unité de cristallisation.

Les premiers et seconds cristaux de paraxylène sont mélangés et introduits dans une zone de fusion 89. Une ligne 90 collecte le courant de paraxylène fondu que l'on introduit dans une colonne de distillation 91 qui délivre, en fond, le paraxylène de très haute pureté et, en tête, du toluène que l'on recycle par une ligne 92 et que l'on mélange à un appoint de toluène apporté par une ligne 95 ou la ligne 18. Le mélange de toluène obtenu est introduit au moins en partie dans chacune des centrifugeuses 82 et 86, comme solvant de lavage.

La figure 7 reprend les mêmes organes et les mêmes références que la figure 6, sauf que pour l'étape de lavage des cristaux, on utilise comme solvant de lavage, du paraxylène fondu très pur récupéré à partir de la ligne 90. En effet, une partie au moins du paraxylène fondu très pur est prélevé par une ligne 91 et introduit dans la première centrifugeuse 82 et dans la seconde centrifugeuse 86 pour laver respectivement les premiers et seconds cristaux. La première liqueur mère et une première liqueur de lavage sont envoyées par la ligne 83 dans la seconde unité de cristallisation 50 tandis que la seconde liqueur mère et la seconde liqueur de lavage sont collectées par la ligne 87 pour être recyclées au moins en partie vers la zone d'adsorption 8.

On a décrit dans cette figure l'utilisation de centrifugeuses 82 et 86 pour séparer les cristaux des liqueurs mère et les laver mais on pourrait aussi bien les remplacer par des colonnes à contre courant, type colonne NIRO. Dans ce cas, les solutions respectives recueillies rassembleraient la liqueur mère et la liqueur de lavage provenant de chaque colonne.

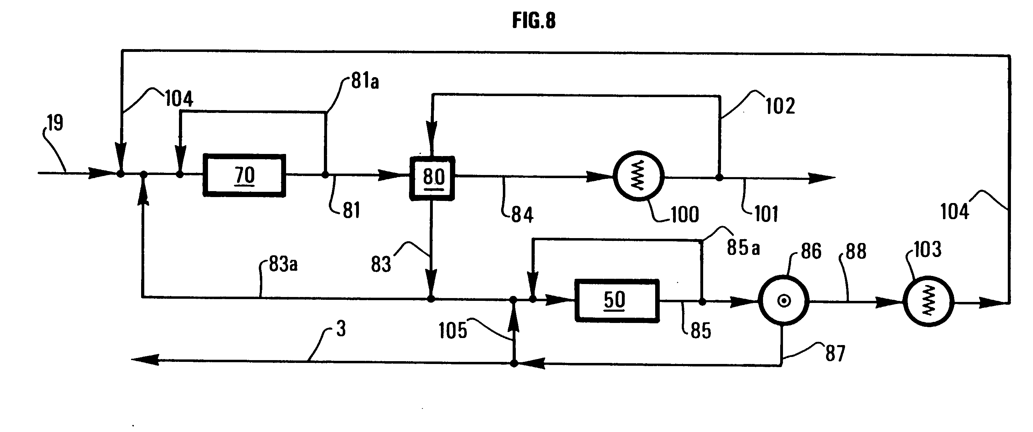

La figure 8, illustre une autre variante du procédé à plusieurs étages de cristallisation, dans laquelle le paraxylène fondu de très haute pureté est recueilli à la sortie de la zone de cristallisation à haute température.

La charge de cristallisation (l'extrait distillé de l'adsorption) est introduite par la ligne 19 dans la première unité de cristallisation 70 qui fonctionne vers 0°C. On récupère par une ligne 81 des cristaux en suspension dans une première liqueur mère qui sont séparés par exemple sur une colonne NIRO 80, les cristaux récupérés par une ligne 84 étant fondus dans une zone de fusion 100. On recueille un courant de paraxylène fondu de très haute pureté par une ligne 101 que l'on prélève en partie grâce à la ligne 102 pour laver les cristaux dans la colonne NIRO. La première liqueur mère qui est soutirée de la colonne NIRO par la ligne 83 est introduite au moins en partie dans une seconde unité de cristallisation 50 opérant à une température de -15°C par exemple. Une autre partie de cette première liqueur mère peut être recyclée par une ligne 83a et mélangée à la charge de la première unité de cristallisation.

De la seconde unité de cristallisation 50, on recueille par un ligne 85 une suspension de seconds cristaux dans une seconde liqueur mère que l'on sépare dans au moins une centrifugeuse 86 ou un filtre rotatif. On récupère par une ligne 87 une seconde liqueur mère que l'on recycle au moins en partie vers l'unité 8 d'adsorption via les zones de traitement à la terre et de distillation, une partie pouvant être recyclée aussi vers la seconde unité de cristallisation 50 par un ligne 105 raccordée à la ligne 87.

Les seconds cristaux, une fois séparés, sont recueillis par une ligne 88 et fondus éventuellement dans une zone de fusion 103. Le paraxylène éventuellement fondu est recyclé par une ligne 104 et mélangé à la charge de la première unité de cristallisation 70 pour être recristallisé à la température de la première cristallisation.

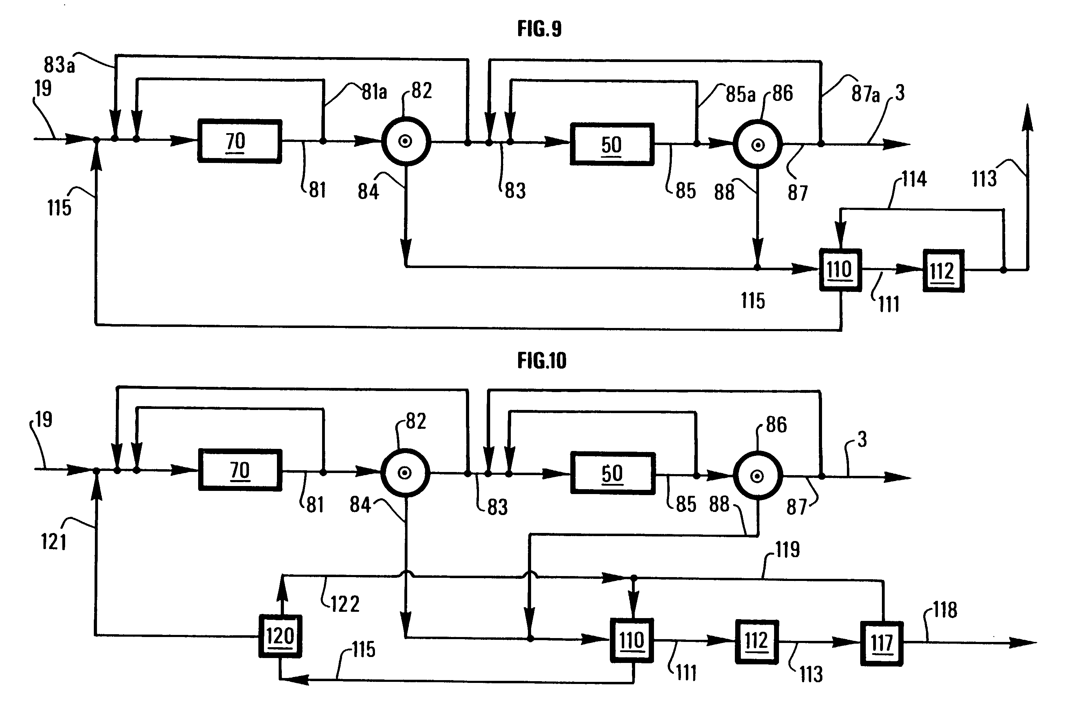

La figure 9 représente une variante préférée du procédé à deux étages de cristallisation opérant avantageusement entre +5 et - 7° C pour le premier et entre -7 et -25° C pour le second.

La charge de cristallisation (l'extrait distillé de l'adsorption) est introduite par la ligne 19 dans une première unité de cristallisation 70. On récupère par une ligne 81 des premières cristaux en suspension dans une première liqueur mère qui sont séparés dans au moins une centrifugeuse 82 ou au moins un filtre rotatif. La première liqueur mère recueillie par la ligne 83 est introduite au moins en partie dans une seconde unité 50 de cristallisation, une autre partie pouvant être recyclée vers la première unité de cristallisation 70. On recueille une seconde suspension de cristaux par une ligne 85 que l'on sépare dans au moins une centrifugeuse ou filtre rotatif 86. Une seconde liqueur mère est soutirée par une ligne 87 et recyclée au moins en partie vers la zone d'adsorption 8 via les zones de traitement à la terre et de distillation, une autre partie pouvant être prélevée et recyclée vers la seconde unité de cristallisation par une ligne 87a raccordée à la ligne 87. Les premiers et seconds cristaux respectivement recueillis par des lignes 84 et 88 sont rassemblés et introduits dans au moins une colonne de lavage 110 type colonne NIRO où ils sont lavés par un solvant de lavage. On recueille des cristaux de paraxylène par une ligne 111 qui sont fondus complètement dans une zone de fusion 112 et on soutire un courant de paraxylène de très haute pureté. Une partie du courant de paraxylène est prélevé par une ligne 114 et introduite comme solvant de lavage dans la colonne 110, La liqueur de lavage recueillie dan la colonne est recyclée au moins en partie vers la première unité de cristallisation.

Selon la figure 10, lorsque le solvant de lavage dans la colonne de lavage est le solvant de désorption (toluène) ou un autre solvant approprié comme le pentane, le courant de paraxylène fondu contenant une mineure partie de solvant peut être distillé dans une unité de distillation 117. Le paraxylène de très haute pureté est recueilli par une ligne 118 tandis que la fraction légère comprenant le solvant de lavage est recyclée dans la colonne NIRO. Enfin, la liqueur de lavage soutirée par la ligne 15 et contenant du solvant est distillée dans une unité de distillation 120, le solvant est recyclé au moins en partie dans la colonne et la liqueur de lavage débarrassée de la majeure partie du solvant est recyclée au moins en partie vers la première unité de cristallisation par la ligne 121.

Il a été mentionné pour une figure donnée le recyclage de la liqueur mère provenant d'un étage A de cristallisation dans ledit étage A ou d'un étage B dans ledit étage B. Il est bien entendu que ces recyclages peuvent s'appliquer pour toutes les figures.

De même, il est connu qu'une suspension de cristaux provenant d'un étage de cristallisation peut être recyclée vers cet étage et ce recyclage peut s'appliquer aussi à toutes les figures (81a et 85a).

On a souvent utilisé dans la description le terme d'une zone de séparation. Bien entendu, il s'agit d'au moins une centrifugeuse ou d'au moins un filtre rotatif ou d'au moins une colonne de lavage à contre courant par un solvant.

La zone de séparation où s'effectue le lavage des cristaux peut comprendre au moins une centrifugeuse ou au moins un filtre rotatif. On a cependant remarqué qu'en utilisant, comme zone de séparation où s'effectue le lavage des cristaux au moins une colonne de lavage à contre courant, type NIRO par exemple et en particulier, avec une partie du paraxylène fondu très pur recueilli, en tant que solvant de lavage, on obtenait d'excellents résultats et on diminuait le coût des utilités.

Comme on l'a schématisé sur la figure 1, la liqueur mère provenant de l'unité de cristallisation 5b est recyclée vers l'unité d'adsorption 8. Dans le cas d'une cristallisation à deux étages ou à plusieurs étages, la liqueur mère provient de l'étage le plus froid de cristallisation après la séparation des cristaux de paraxylène (ligne 53, figure 5, ligne 87, figure 7 à 9). Les impuretés qui circulent dans la boucle du dispositif adsorption, cristallisation, isomérisation peuvent être des hydrocarbures oléfiniques, ainsi que des hydrocarbures paraffiniques et naphténiques ou autres composés oxygénés. Elles peuvent provenir notamment, aussi bien de la charge à traiter qui provient d'un réformage catalytique que de l'isomérisation. Ces impuretés circulent donc et peuvent se retrouver dans toutes les fractions et notamment dans l'extrait et donc dans la liqueur mère résultant de l'étape de cristallisation. Cette liqueur mère peut être introduite par une ligne 32 reliée à la ligne 3 et à la ligne 53 ou 87 dans au moins un réacteur 26 de traitement à la terre, avantageusement deux, disposé en amont et relié par une ligne 27 à l'unité d'adsorption 8. A cette ligne 32 peuvent être raccordées la ligne 1 contenant la charge à traiter et la ligne 2 contenant l'isomérat, les trois flux étant ainsi traités en mélange dans un réacteur unique 26.

Selon une autre variante, la charge 1 peut avoir été prétraitée dans un autre réacteur de traitement à la terre (non indiqué sur la figure). Il en est de même pour l'isomérat 2 qui peut aussi avoir été initialement prétraité après son passage dans l'unité de distillation 23.

Selon une variante préférée, la liqueur mère 3 peut être introduite directement dans la ligne 22 conduisant à l'unité de distillation 23 de l'isomérat, avant d'être traitée, en mélange avec l'isomérat distillé, dans le réacteur 26 de traitement à la terre. Cette variante permet d'éliminer sensiblement tous les composés les plus volatils non seulement de l'isomérat mais de la liqueur mère.

Lorsque l'unité de distillation est réglée pour délivrer aussi, en fond, une fraction supplémentaire contenant la majorité des composés lourds (hydrocarbures en C9+, aldéhydes, cétones), le traitement à la terre du mélange distillé comprenant l'isomérat et la liqueur mère s'en trouve substantiellement amélioré.

Une partie de la liqueur mère peut aussi être recyclée par une ligne 31 à l'effluent 27 du réacteur 26.

L'effluent du réacteur de traitement à la terre et éventuellement la liqueur mère de cristallisation 31 pouvant contenir encore des hydrocarbures lourds tels que des hydrocarbures à neuf atomes de carbone sont introduits par une ligne 27 dans une colonne à distiller 28 qui délivre, en fond de colonne (ligne 29), les impuretés indésirables et en tête un distillat correspondant à la coupe C₈ purifiée, que l'on introduit par la ligne 4 à l'unité d'adsorption 8. Une partie de la liqueur mère peut aussi être introduite dans la ligne 4 par une ligne 30.

Ces divers recyclages peuvent être combinés entre eux, lorsqu'il s'agit, par exemple, de réutiliser des équipements existants pour la distillation 23, le traitement à la terre 26 ou la distillation 28 et lorsque l'un de ces équipements est déjà opéré à son débit maximum ou bien encore lorsque l'on cherche à faire baisser la teneur en une impureté dans la boucle sans chercher à l'éliminer totalement. En d'autres termes, la liqueur mère de l'unité de cristallisation (étage le plus froid), véhiculée par la ligne 3 peut être en partie recyclée vers l'unité d'adsorption 8 soit directement au moyen de la ligne 30, soit indirectement au moyen des lignes 31, 32 ou 33.

De manière à contenir la teneur en constituants de volatilité intermédiaire du toluène (solvant de desorption) à un niveau tolérable, par exemple inférieur à 5 %, on effectue au moins une purge de toluène pollué par lesdits constituants au moyen d'une ligne 35 reliée soit à la ligne 17, soit à la ligne 14 soit à la ligne 11 collectant l'ensemble du solvant recyclé vers l'unité d'adsorption 8.

De plus, on peut effectuer une purge de la liqueur mère issue de la cristallisation si la teneur en constituants de volatilité intermédiaire y est trop forte. Cette purge est réalisée au moyen de la ligne 34 raccordée à la ligne 3.

La purge de toluène peut être compensée par un appoint de toluène. Étant donné que les sources les plus importantes de coupe C₈ aromatique (ligne 1a) proviennent du reformage catalytique, de la dismutation (disproportionnation) du toluène en benzène et xylène et de la transalkylation toluène - C₉ aromatique et que les effluents de ces unités dont il proviennent sont en partie généralement purifiés dans un train d'unités de distillation dont la colonne 28 peut faire partie, on peut utiliser au moins en partie comme source d'appoint de toluène, soit celui produit en tête (ligne 42) d'une colonne de distillation 40 du toluène en avant du réacteur 26 contenant la terre soit celui qui résulte du by-pass par une ligne 1b d'une partie au moins de la charge (ligne 1) que l'on mélange à l'effluent de fond de la colonne 40 soit celui qui est introduit dans la charge purifiée (ligne 1) par le déréglage de la colonne 40 qui permet de laisser passer dan la coupe C8 la proportion voulue de toluène.

## Revendications

1. Procédé de séparation et de récupération du paraxylène à partir d'une charge pouvant contenir du métaxylène, ce procédé comprenant en combinaison une opération d'adsorption et une opération de cristallisation, **caractérisé en ce que** au moins une partie de la charge et/ou de la liqueur mère résultant de la cristallisation, subit une opération de traitement à la terre, ladite terre étant choisie dans le groupe des silicoaluminates naturels activés ou non, la température étant de 100 à 300 °C, la pression de 3 à 100 bar et la vitesse horaire spatiale de 1 à 8.

2. Procédé selon la revendication 1, comportant en combinaison en plus des opérations d'adsorption et de cristallisation une étape d'isomérisation, délivrant un isomérat **caractérisé en ce que** au moins une partie de l'isomérat est traitée à la terre.

3. Procédé selon la revendication 2, **caractérisé en ce que** une partie au moins de la charge et une partie au moins de l'isomérat subissent chacune une opération de traitement à la terre.

4. Procédé de séparation et de récupération de paraxylène contenu dans une charge d'hydrocarbures comprenant des hydrocarbures aromatiques en C₈, procédé dans lequel notamment :
• on met en contact dans au moins une zone d'adsorption (8), ladite charge (1) contenant au moins du métaxylène, du paraxylène avec au moins un adsorbant en présence d'au moins un solvant de désorption approprié dans des conditions d'adsorption telles qu'on obtient une première fraction contenant du solvant, du métaxylène et éventuellement de l'éthylbenzène et/ou de l'orthoxylène et une seconde fraction contenant du solvant du paraxylène substantiellement enrichi ;
• on distille (12) la première fraction en vue de récupérer un mélange métaxylène et éventuellement éthylbenzène, et/ou orthoxylène ;
• on isomérise au moins en partie ledit mélange dans des conditions appropriées dans au moins une zone d'isomérisation (21) et l'on récupère un isomérat (2) que l'on recycle au moins en partie vers la zone d'adsorption ;
• on distille (16) ladite deuxième fraction en vue d'obtenir du paraxylène substantiellement enrichi et on procède à une cristallisation du paraxylène substantiellement enrichi avec obtention d'une part d'une liqueur mère que l'on recycle (3) au moins en partie vers l'étape d'adsorption et d'autre part des cristaux de paraxylène.
le procédé étant **caractérisé en ce qu'**on fait circuler au moins une partie choisie parmi au moins en partie la charge (1), au moins en partie l'isomérat (2) et au moins en partie la liqueur mère (3) (32) dans au moins une zone (26) de traitement à la terre ou matériau équivalent, ladite terre étant choisie dans le groupe des silicoaluminates naturels activés ou non, la température étant de 100 à 300 °C, la pression de 3 à 100 bar et la vitesse horaire spatiale de 1 à 8, et l'on récupère un premier effluent (27) que l'on introduit au moins en partie dans la zone d'adsorption (8) pour produire les dites première et deuxième fractions de l'étape soutirées de la zone d'adsorption.

5. Procédé selon la revendication 4, dans lequel on lave les cristaux de paraxylène avec un solvant de lavage approprié dans au moins une zone de lavage et l'on récupère des cristaux de paraxylène à un très grand degré de pureté.

6. Procédé selon l'une des revendications 4 à 5, dans lequel on fait circuler une partie au moins de la charge (1) et de l'isomérat dans au moins une zone de traitement à la terre.

7. Procédé selon l'une des revendications 4 à 6, dans lequel on fait circuler une partie au moins de la charge, de l'isomérat (2) et de la liqueur mère dans une ou plusieurs zones de traitement à la terre.

8. Procédé selon l'une des revendications 4 à 7 **caractérisé en ce que** la zone d'adsorption est opérée en lit mobile simulé.

9. Procédé selon l'une des revendications 4 à 8, dans lequel au moins une partie de la liqueur mère (3) (33) est introduite dans une colonne de distillation (23) en aval de la zone (21) d'isomérisation, on distille l'isomérat et la liqueur mère, on obtient une fraction de tête contenant des composés légers et une fraction, contenant un mélange de ladite liqueur mère et d'un isomérat distillés, qui est introduite ensuite dans la zone (26) de traitement à la terre et l'on récupère un second effluent.

10. Procédé selon la revendication 9, dans lequel, en outre, une fraction de queue contenant des composés lourds est soutirée de la colonne de distillation (23).

11. Procédé selon l'une des revendications 4 à 10, dans lequel une partie de la liqueur mère (31) est mélangée aux premier ou second effluent de la zone de traitement à la terre et l'on récupère un troisième effluent.

12. Procédé selon l'une des revendications 4 à 11, dans lequel le premier, le second ou le troisième effluent de la zone de traitement à la terre est distillé dans au moins une colonne (28) à distiller (colonne dite rerun) qui délivre une fraction de queue (29) contenant des composés lourds et une fraction de tête (4) qui est introduite dans la zone d'adsorption (8).

13. Procédé selon l'une des revendications 1 à 12 dans lequel le traitement à la terre est effectué à une la température de 160 à 230°C, à une pression de 4 à 20 bar, et à une vitesse horaire spatiale de 1 à 4.

14. Procédé selon l'une des revendications 4 à 13 dans lequel au moins une partie de la liqueur mère est purgée avant d'être introduite dans la zone de traitement à la terre.

15. Procédé selon l'une des revendications 4 à 14 dans lequel le solvant de désorption résultant des étapes (b) et (d) est purgé au moins en partie avant d'être recyclé et on compense la purge du solvant par un apport de solvant frais, soit dans la charge soit en amont de la zone d'adsorption.

16. Procédé selon l'une des revendications 4 à 15, dans lequel on effectue une cristallisation (50) à un seul étage, du paraxylène de l'étape (d), on récupère une suspension de cristaux (51) dans la liqueur mère, on sépare les cristaux de la liqueur mère dans une zone (52) de séparation, on lave les cristaux obtenus avec le solvant de lavage, on récupère une liqueur de lavage, on recycle une partie au moins de la liqueur mère (53) et une partie au moins de la liqueur de lavage et on fond (58) complètement les cristaux de paraxylène de façon à obtenir un courant liquide de paraxylène fondu.

17. Procédé selon la revendication 16, dans lequel la zone de séparation et de lavage est au moins une colonne (80) de lavage à contre courant dans laquelle on introduit comme solvant de lavage (59b) une partie au moins du courant liquide de paraxylène fondu et dans lequel la liqueur mère et la liqueur de lavage ne sont qu'une même liqueur (53) que l'on recycle au moins en partie vers la zone d'adsorption (8) et éventuellement en partie vers la zone de cristallisation (50).

18. Procédé selon la revendication 16, dans lequel la zone de séparation et de lavage est au moins une centrifugeuse ou au moins un filtre rotatif dans lequel on introduit comme solvant de lavage, une partie au moins du courant liquide de paraxylène fondu et dans lequel la liqueur mère et la liqueur de lavage sont recyclées vers la zone d'adsorption et éventuellement en partie vers la zone de cristallisation.

19. Procédé selon la revendication 16 dans lequel le solvant de lavage est le solvant de désorption, on distille (60) le courant liquide de paraxylène de façon à récupérer dans une fraction de tête, du solvant (56) de lavage que l'on recycle au moins en partie dans la zone de lavage (52) des cristaux, et dans une fraction de queue du paraxylène liquide (61) de très haute pureté, soit on recycle la liqueur mère (53) comprenant du solvant de lavage vers la zone d'adsorption (8) et éventuellement en partie vers la zone de cristallisation (50), après l'avoir éventuellement distillée, lorsque la zone de lavage est une colonne de lavage à contre courant, soit on recycle la liqueur mère (53) vers la zone (8) d'adsorption et éventuellement en partie vers la zone de cristallisation (50) et la liqueur de lavage dans une zone de distillation selon l'étape (b) lorsque la zone de lavage est une centrifugeuse ou un filtre rotatif (52).

20. Procédé selon la revendication 16, dans lequel le solvant de lavage est un solvant autre que le courant liquide de paraxylène fondu et le solvant de désorption, on distille le courant liquide de paraxylène fondu de façon à récupérer, en tête , du solvant que l'on recycle au moins en partie dans la zone de lavage des cristaux et en fond, du paraxylène liquide de très haute pureté et dans lequel soit on recycle la liqueur mère comprenant la liqueur de lavage vers la zone d'adsorption et éventuellement en partie vers la zone de cristallisation, après l'avoir distillée, le solvant étant recyclé dans la zone de lavage, lorsque la zone de lavage est une zone de lavage à contre courant, soit on recycle la liqueur mère vers la zone d'adsorption et éventuellement en partie vers la zone de cristallisation et la liqueur de lavage, après l'avoir distillée, vers la zone d'adsorption et éventuellement en partie vers la zone de cristallisation, le solvant étant recyclé à la zone de lavage, lorsque la zone de lavage est une centrifugeuse ou un filtre rotatif.

21. Procédé selon l'une des revendications 4 à 15, dans lequel on effectue (figures 4 et 5) une première cristallisation du paraxylène de l'étape (d) dans une première zone (50) de cristallisation à une température T₁, on récupère une suspension (51) de premiers cristaux de paraxylène dans une première liqueur mère, on sépare les premiers cristaux de la première liqueur mère dans une première zone (52) de séparation, on recycle une partie au moins de la première liqueur mère vers la zone (8) d'adsorption, on fond (58) les premiers cristaux séparés et on les cristallise dans une deuxième zone (70) de cristallisation à une température T2 supérieure à T1, on récupère une suspension (71) de seconds cristaux de paraxylène dans une seconde liqueur mère, on sépare les seconds cristaux de la seconde liqueur mère dans une seconde zone (72) de séparation, on lave les seconds cristaux avec le solvant de lavage approprié dans la seconde zone de séparation, on récupère une liqueur de lavage, on recycle au moins en partie la seconde liqueur mère (73) et une partie au moins de la liqueur de lavage vers la première zone de cristallisation et éventuellement en partie vers la seconde zone de cristallisation et on fond (76) complètement les seconds cristaux de paraxylène de façon à obtenir un courant (77) liquide de paraxylène fondu.

22. Procédé selon la revendication 21, dans lequel le solvant de lavage est une partie du courant liquide de paraxylène fondu et la liqueur de lavage et la seconde liqueur mère ne sont qu'une même liqueur recyclée au moins en partie vers la première zone de cristallisation et éventuellement en partie vers la seconde zone de cristallisation.

23. Procédé selon la revendication 21, dans lequel le solvant de lavage est le solvant de désorption, par exemple le toluène et dans lequel on distille (60) le courant de paraxylène fondu, de façon à récupérer dans une fraction de tête du solvant de lavage que l'on recycle au moins en partie dans la seconde zone (72) de séparation, et dans une fraction de queue, du paraxylène liquide de très haute pureté et soit on distille éventuellement la seconde liqueur mère contenant la liqueur de lavage avant de la recycler lorsque. la seconde zone de séparation est une colonne de lavage à contre courant, soit on distille la liqueur de lavage avant de la recycler vers la première unité de cristallisation et/ou vers la seconde unité de cristallisation lorsque la seconde zone de séparation est une centrifugeuse ou un filtre rotatif.

24. Procédé selon la revendication 21, dans lequel le solvant de lavage est un solvant autre que le courant de paraxylène fondu et le solvant de désorption, on distille (60) le courant de paraxylène fondu provenant de la suspension de seconds cristaux de façon à récupérer dans une fraction de tête, du solvant de lavage que l'on recycle au moins en partie dans la seconde zone de séparation et dans une fraction de queue, du paraxylène liquide de très haute pureté et soit on distille la liqueur mère comprenant la liqueur de lavage avant de la recycler vers la première zone de cristallisation et éventuellement en partie vers la seconde zone de cristallisation lorsque la seconde zone de séparation est une colonne à contre-courant, soit on recycle la liqueur mère vers la première zone de cristallisation et éventuellement en partie vers la seconde zone de cristallisation, on distille la liqueur de lavage avant de la recycler vers la première zone de cristallisation et éventuellement en partie vers la seconde zone de cristallisation, lorsque la seconde zone de séparation est une centrifugeuse ou un filtre rotatif.

25. Procédé selon l'une des revendications 4 à 15, dans lequel on effectue (figures 6 et 7) une première cristallisation du paraxylène de l'étape (d) dans une première zone (70) de cristallisation à une température T1, on récupère une suspension (81) de premiers cristaux de paraxylène dans une première liqueur mère, on sépare les premiers cristaux de la première liqueur mère dans une première zone (82) de séparation, on lave éventuellement les premiers cristaux avec le solvant de lavage dans la première zone de séparation, on cristallise une partie au moins de la première liqueur mère dans une seconde zone (50) de cristallisation à une température T2 inférieure à la température T1, on récupère une suspension (85) de seconds cristaux de paraxylène dans une seconde liqueur mère, on sépare les seconds cristaux de la seconde liqueur mère dans une seconde zone (86) de séparation, on lave les seconds cristaux avec le solvant de lavage approprié dans la zone de séparation, en récupère une seconde liqueur mère (87) que l'on recycle au moins en partie vers la zone (8) d'adsorption, on mélange les premiers et les seconds cristaux de paraxylène, on les fond (89) complètement et on récupère un courant (90) de paraxylène fondu.

26. Procédé selon la revendication 25 dans lequel un partie au moins du courant de paraxylène fondu est prélevée et utilisée comme solvant de lavage dans la première zone (82) de séparation et dans la seconde zone (86) de séparation.

27. Procédé selon la revendication 25, dans lequel le solvant de lavage est le solvant de désorption, par exemple le toluène et dans lequel on distille le courant de paraxylène fondu, on récupère une fraction de tête contenant du solvant de lavage qui est recyclé au moins en partie vers la première et la deuxième zone de séparation et une fraction de queue contenant du paraxylène liquide de très haute pureté.

28. Procédé selon l'une des revendications 5 à 15, dans lequel on effectue (figure 8) une première cristallisation du paraxylène de l'étage (d) et de cristaux éventuellement fondus recyclés provenant d'une seconde cristallisation ci-après, dans une première zone (70) de cristallisation à une température T1, on récupère une suspension (81) de cristaux de paraxylène dans une première liqueur mère, on sépare les cristaux de la première liqueur mère dans une première zone (82) de séparation, on lave lesdits cristaux dans ladite zone, on les fond (100) et on récupère un courant de paraxylène fondu, on cristallise une partie au moins de la première liqueur mère (83) dans une seconde zone (50) de cristallisation à une température T2 inférieure à la température T1, on récupère une suspension de seconds cristaux de paraxylène dans une seconde liqueur mère, on sépare les seconds cristaux de la seconde liqueur mère dans une seconde zone (86) de séparation, on récupère une seconde liqueur mère (87) que l'on recycle au moins une partie vers la zone d'adsorption, on fond (103) éventuellement les seconds cristaux et on recycle les cristaux éventuellement fondus vers la première zone de cristallisation pour les recristalliser avec le paraxylène de l'étape (d) à la température T1.

29. Procédé selon l'une des revendications 4 à 15, dans lequel on effectue (figure 8) une première cristallisation du paraxylène de l'étage (d) et de cristaux partiellement fondus recyclés provenant d'une seconde cristallisation ci-après, dans une première zone (70) de cristallisation à une température T1, on récupère une suspension (81) de cristaux de paraxylène dans une première liqueur mère, on sépare les cristaux de la première liqueur mère dans une première zone (82) de séparation, on lave lesdits cristaux dans ladite zone, on les fond (100) et on récupère un courant de paraxylène fondu, on cristallise une partie au moins de la première liqueur mère (83) dans une seconde zone (50) de cristallisation à une température T2 inférieure à la température T1, on récupère une suspension de seconds cristaux de paraxylène dans une seconde liqueur mère, on sépare les seconds cristaux de la seconde liqueur mère dans une seconde zone (86) de séparation, on récupère une seconde liqueur mère (87) que l'on recycle au moins une partie vers la zone d'adsorption, on fond (103) partiellement les seconds cristaux et on recycle les cristaux en suspension vers la première zone de cristallisation pour les recristalliser avec le paraxylène de l'étape (d) à la température T1.

30. Procédé selon les revendications 28 et 29 dans lequel une partie au moins du courant de paraxylène fondu provenant de la première zone de séparation est utilisée comme solvant de lavage .

31. Procédé selon les revendications 28 et 29, dans lequel le solvant de lavage est le solvant de désorption et dans lequel on distille le courant de paraxylène fondu provenant de la première zone de cristallisation et l'on récupère dans une fraction de tête du solvant de lavage que l'on recycle au moins en partie dans la première zone de séparation et dans une fraction de queue, du paraxylène liquide de très haute pureté.

32. Procédé selon l'une des revendications 4 à 15, dans lequel on effectue (figure 9 et 10) une première cristallisation du paraxylène de l'étape (d) dans une première zone de cristallisation à une température T1, on récupère une suspension de premiers cristaux de paraxylène dans une première liqueur mère, on sépare les premiers cristaux de la première liqueur mère dans une première zone (82) de séparation, on cristallise une partie au moins de la première liqueur mère dans une seconde zone (50) de cristallisation à une température T₂ inférieure à T₁, on sépare les seconds cristaux de la seconde liqueur mère dans une seconde zone (86) de séparation, on récupère la seconde liqueur mère que l'on recycle au moins une partie vers la zone d'adsorption (8), on récupère les premiers cristaux et les seconds cristaux et on les lave dans au moins une zone (110) de séparation et de lavage à contre courant avec le solvant de lavage approprié, on récupère d'une part une liqueur (115) de lavage qui est recyclée au moins en partie vers la première zone (70) de cristallisation, on récupère d'autre part des cristaux de paraxylène, on les fond complètement dans une zone de fusion (112) et on recueille un courant de paraxylène fondu.

33. Procédé selon la revendication 32, dans lequel une partie du courant de paraxylène fondu est prélevée et utilisée comme solvant de lavage.

34. Procédé selon la revendication 32 dans lequel le solvant de lavage est le solvant de désorption, on distille le courant de paraxylène fondu, on recueille du solvant de lavage que l'on recycle au moins en partie dans la zone de lavage, on distille la liqueur de lavage avant de la recycler vers la première zone de cristallisation et on recycle au moins en partie le solvant de lavage dans la zone de lavage.

35. Procédé selon l'une des revendications 21 à 34 dans lequel une partie de la première liqueur mère est recyclée vers la première zone de cristallisation et une partie de la seconde liqueur mère vers la seconde zone de cristallisation.

36. Procédé selon l'une des revendications 5 à 35 dans lequel la zone de séparation où s'effectue le lavage des cristaux comprend au moins une colonne de lavage à contre courant.

37. Procédé selon l'une des revendications 5 à 35 dans lequel la zone de séparation où s'effectue le lavage des cristaux comprend au moins une centrifugeuse ou au moins un filtre rotatif.

38. Procédé selon l'une des revendications 5 à 37 dans lequel ledit solvant de désorption est le toluène et dans lequel on fait circuler une partie de la charge dans une colonne de distillation (40) pour en soutirer une partie au moins du toluène (32) et l'on introduit ledit toluène comme appoint de solvant de désorption.

39. Procédé selon l'une des revendications 4 à 38, **caractérisé en ce que** le solvant de desorption est soit du toluène soit du paradiéthylbenzène.

## Claims

1. Process for separating and recovering paraxylene from a charge that can contain metaxylene, this process comprising in combination an adsorption operation and a crystallization operation, **characterized in that** at least a portion of the charge and/or of the mother liquor resulting from the crystallization, undergoes a clay operation, said clay being selected from the group of the natural, activated or not silicoaluminates, the temperature being 100 to 300°C, the pressure being 3 to 100 bar and the hourly space velocity being 1 to 8.

2. Process according to claim 1, comprising, in combination, besides the adsorption and crystallization operations, an isomerization step delivering an isomerate, wherein at least a portion of the isomerate is clay-treated.

3. Process according to claim 2, wherein at least a portion of the charge and at least a portion of the isomerat each undergo a clay treatment operation.

4. Process for separating and recovering paraxylene contained in a charge of hydrocarbons comprising C₈-aromatic hydrocarbons, a process in which in particular :
• In at least one adsorption zone (8), said charge (1) containing at least metaxylene, paraxylene is put in contact with at least one adsorbent in the presence of at least one suitable desorption solvent under adsorption conditions such that there is obtained a first fraction containing solvent, metaxylene and optionally ethylbenzene and/or orthoxylene and a second fraction containing solvent of the substantially enriched paraxylene ;
• The first fraction is distilled (12) to recover a mixture of metaxylene and optionally ethylbenzene, and/or orthoxylene ;
• Said mixture is isomerized at least in part under suitable conditions in at least one isomerization zone (21) and an isomerate (2) is recovered that is recycled at least partially to the adsorption zone ;
• Said second fraction is distilled (16) to obtain substantially enriche paraxylene and a crystallization of the substantially enriched paraxylene is performed obtaining, on the one hand, a mother liquor that is at least partially recycled (3) to the adsorption step and, on the other hand, paraxylene crystals.
The process being **characterized in that** at least one portion selected from at least in part the charge (1), at least in part the isomerate (2) and at least in part the mother liquor (3) (32), is made to circulate in at least one clay or equivalent material treatment zone, said clay being selected from the group of the natural, activated or not silicoaluminates, the temperature being 100 to 300°C, the pressure being 3 to 100 bar and the hourly space velocity being 1 to 8 and a first effluent (27) is recovered that is introduced at least in part into the adsorption zone (8) to produce said first and second fractions of the step that are drawn off from the adsorption zone and a first effluent (27) is recovered that is introduced at least in part into the adsorption zone (8) to produce said first and second fractions of the step that are drawn off from the adsorption zone.

5. Process according to claim 4, wherein the paraxylene crystals are washed with an appropriate washing solvent in at least one washing zone and paraxylene crystals having a very high degree of purity are recovered.

6. Process according to one of claims 4 to 5, wherein at least a portion of the charge (1) and of the isomerate is made to circulate in at least one clay treatment zone.

7. Process according to one of claims 4 to 6, wherein at least a portion of the charge, the isomerate (2) and the mother liquor is made to circulate in one or more clay treatment zones.

8. Process according to one of claims 4 to 7, wherein the adsorption zone is effected in a simulated moving bed.

9. Process according to one of claims 4 to 8, wherein at least a portion of the mother liquor (3) (33) is introduced into a distilled column (23) downstream from isomerization zone (21), the isomerate and the mother liquor are distilled, there is obtained a fraction at the top containing light compounds and a fraction, containing a mixture of said distilled mother liquor and a distilled isomerate, which is then introduced into the clay treatment zone (26) and a second effluent s recovered.

10. Process according to claims 9, wherein, in addition, a bottom fraction containing heavy compounds is drawn off from distilling column (23).

11. Process according to one of claims 4 to 10, wherein a portion of the mother liquor (31) is mixed with the first or second effluent of the clay treatment zone and a third effluent is recovered.

12. Process according to one of claims 4 to 11, wherein the first, second or third effluent from the clay treatment zone is distilled in at least one distilling column (28) (a so-called rerun column) which delivers a bottom fraction (29) containing heavy compounds and a top fraction (4) which is introduced into the adsorption zone (8).

13. Process according to one of claims 1 to 12, wherein the clay treatment is carried out at a temperature from 160 to 230°C, the pressure is from 4 to 20 bar, and the hourly space velocity 1 to 4.

14. Process according to one of claims 4 to 13, wherein at least portion of the mother liquor is purged before being introduced into the clay treatment zone.

15. Process according to one of claims 4 to 14, wherein the desorption solvent resulting from steps (b) and (d) is purged at least in part before being recycled and the purge of the solvent is compensated for by an addition of fresh solvent, either into the charge or upstream from the adsorption zone.

16. Process according to one of claims 4 to 15, wherein a crystallization (50) of the paraxylene of step (d) is performed in a single stage, a suspension of crystals (51) in the mother liquor is recovered, the crystals are separated from the mother liquor in a separation zone (52), the crystals obtained are washed with the washing solvent, a washing liquor is recovered, at least a portion of the mother liquor (53) and at least a portion of the washing liquor are recycled and the paraxylene crystals are melted (58) completely so as to obtain a liquid stream of melted paraxylene.

17. Process according to claim 16, wherein the zone for separating and washing is at least one countercurrent washing column (52) into which at least a portion of the liquid stream of melted paraxylene is introduced as washing solvent (59b) and in which the mother liquor and the washing liquor are one and the same liquor (53) that is recycled at least in part to the adsorption zone (8) and optionally in part to the crystallization zone (50).

18. Process according to claim 16, wherein the zone for separating and washing is at least one centrifuge or at least one rotary filter in which at least a portion of the liquid stream of melted paraxylene is introduced as washing solvent and in which the mother liquor and the washing liquor are recycled to the adsorption zone and optionally in part to the crystallization zone.

19. Process according to claim 16, wherein the washing solvent is the desorption solvent, the liquid stream of paraxylene is distilled (60) so as to recover, in a fraction at the top, washing solvent (56) that is recycled at least in part in the washing zone (52) of the crystals, and, in a bottom fraction, liquid paraxylene (61) of very high purity,
either the mother liquor (53) comprising washing solvent is recycled to the adsorption zone (8) and optionally in part to the crystallization zone (50), after having been distilled, when the washing zone is a countercurrent washing column, or the mother liquor (53) is recycled to adsorption zone (8) and optionally in part to the crystallization zone (50) and the washing liquor into a distillation zone according to step (b) when the washing zone is a centrifuge or a rotary filter (52).

20. Process according to claim 16, wherein the washing solvent is a solvent other than the liquid stream of melted paraxylene and the desorption solvent, the liquid stream of melted paraxylene is distilled so as to recover, at the top, washing solvent that is recycled at least in part in the washing zone of the crystals and, at the bottom, liquid paraxylene of very high purity and in which either the mother liquor comprising the washing liquor is recycled to the adsorption zone and optionally in part to the crystallization zone, after having been distilled, the solvent being recycled in the washing zone, when the washing zone is a countercurrent washing zone, or the mother liquor is recycled to the adsorption zone and optionally in part to the crystallization zone and the washing liquor, after having been distilled, is recycled to the adsorption zone and optionally in part to the crystallization zone, the solvent being recycled to the washing zone, when the washing zone is a centrifuge or a rotary filter.

21. Process according to one of claims 4 to 15, wherein a first crystallization of the paraxylene of step (d) is performed (figures 4 and 5) in a first crystallization zone (50) at a temperature T1, a suspension (51) of first paraxylene crystals in a first mother liquor is recovered, the first crystals are separated from the first mother liquor in a first separation zone (52), at least a portion of the first mother liquor is recycled to the adsorption zone (8), the separated first crystals are completely melted (58) and they are crystallized in a second crystallization zone (70) at a temperature T2 that is higher than T1, a suspension (71) of second paraxylene crystals in a second mother liquor is recovered, the second crystals are separated from the second mother liquor in a second separation zone (72), the second crystals are washed with the suitable washing solvent in the second separation zone, a washing liquor is recovered, the second mother liquor (73), at least in part, and at least a portion of the washing liquor are recycled to the first crystallization zone and optionally in part to the second crystallization zone and the second paraxylene crystals are melted (76) completely so as to obtain a liquid stream (77) of melted paraxylene.

22. Process according to claim 21, wherein the washing solvent is a portion of the liquid stream of melted paraxylene and the washing liquor and the second mother liquor are one and the same liquor that is recycled to the first crystallization zone and optionally in part to the second crystallization zone.

23. Process according to claim 21, wherein the washing solvent is the desorption solvent, for example toluene, and wherein the stream of melted paraxylene is distilled (60) so as to recover, in a top fraction, washing solvent that is recycled at least in part in the second separation zone (72), and, in a bottom fraction, liquid paraxylene of very high purity, and either the second mother liquor containing the washing liquor is distilled before recycling it when the second separation zone is a countercurrent washing column, or the washing liquor is distilled before recycling it to the first crystallization unit and/or to the second crystallization unit when the second separation zone is a centrifuge or a rotary filter.

24. Process according to claim 21, wherein the washing solvent is a solvent other than the stream of melted paraxylene and the desorption solvent, the stream of melted paraxylene coming from the suspension of second crystals is distilled (60) so as to recover, in a top fraction, washing solvent that is recycled at least in part in the second separation zone and, in a bottom fraction, liquid paraxylene of very high purity, and either the mother liquor comprising the washing liquor is distilled before recycling it to the first crystallization zone and optionally in part to the second crystallization zone when the second separation zone is a countercurrent column, or the mother liquor is recycled to the first crystallization zone and optionally in part to second crystallization zone, the washing liquor is distilled before recycling it to the first crystallization zone and optionally in part to the second crystallization zone, when the second separation zone is a centrifuge or a rotary filter.

25. Process according to one of claims 5 to 15, wherein a first crystallization of the paraxylene of step (d) is performed (figures 6 and 7) in a first crystallization zone (70) at a temperature T1, a suspension (81) of first paraxylene crystals in a first mother liquor is recovered, the first crystals are separated from the first mother liquor in a first separation zone (82), optionally the first crystals are washed with the washing solvent in the first separation zone, at least a portion of the first mother liquor is crystallized in a second crystallization zone (50) at a temperature T2 that is lower than temperature T1, a suspension (85) of second paraxylene crystals in a second mother liquor is recovered, the second crystals are separated from the second mother liquor in a second separation zone (86), the second crystals are washed with the suitable washing solvent in the separation zone, a second mother liquor (87) is recovered that is recycled at least in part to the adsorption zone (8), the first and second paraxylene crystals are mixed, they are melted (89) completely and a stream (90) of melted paraxylene is recovered.

26. Process according to claim 25, wherein at least a portion of the stream of melted paraxylene is drawn off and used as washing solvent in the first separation zone (82) and in the second separation zone (86).

27. Process according to claim 25, wherein the washing solvent is the desorption solvent, for example toluene, and wherein the stream of melted paraxylene is distilled, a top fraction containing washing solvent which is recycled at least in part to the first and second separation zones, and a bottom fraction containing liquid paraxylene of very high purity, are recovered.

28. Process according to one of claims 5 to 15, wherein a first crystallization of the paraxylene of stage (d) and of optionally melted recycled crystals coming from a second crystallization afterward is performed (figure 8) in a first crystallization zone (70) at a temperature T1, a suspension (81) of paraxylene crystals in a first mother liquor is recovered, the crystals are separated from the first mother liquor in a first separation zone (82), said crystals are washed in said zone, they are melted (100) and a stream of melted paraxylene is recovered, at least a portion of the first mother liquor (83) is crystallized in a second crystallization zone (50) at a temperature T2 that is lower than temperature T1, a suspension of second paraxylene crystals in a second mother liquor is recovered, the second crystals are separated from the second mother liquor in a second separation zone (86), a second mother liquor (87) is recovered that is recycled at least in part to the adsorption zone, optionally the second crystals are melted (103) and the optionally melted crystals are recycled to the first crystallization zone to recrystallize them with the paraxylene of step (d) at temperature T1.

29. Process according to one of claims 4 to 15, wherein a first crystallization of the paraxylene of stage (d) and of partially melted recycled crystals coming from a second crystallization afterward is performed (figure 8) in a first crystallization zone (70) at a temperature T1, a suspension (81) of paraxylene crystals in a first mother liquor is recovered, the crystals are separated from the first mother liquor in a first separation zone (82), said crystals are washed in said zone, they are melted (100) and a stream of melted paraxylene is recovered, . at least a portion of the first mother liquor (83) is crystallized in a second crystallization zone (50) at a temperature T2 that is lower than temperature T1, a suspension of second paraxylene crystals in a second mother liquor is recovered, the second crystals are separated from the second mother liquor in a second separation zone (86), a second mother liquor (87) is recovered that is recycled at least in part to the adsorption zone, the second crystals are partially melted (103) and the crystals in a slurry are recycled to the first crystallization zone to recrystallize them with the paraxylene of step (d) at temperature T1.

30. Process according to the claims 28 and 29, wherein at least a portion of the stream of melted paraxylene coming from the first separation zone is used as washing solvent.

31. Process according to the claims 28 and 29, wherein the washing solvent is the desorption solvent and wherein the stream of melted paraxylene coming from the first crystallization zone is distilled and there is recovered, in a top fraction, washing solvent that is recycled at least in part in the first separation zone and, in a bottom fraction, liquid paraxylene of very high purity.

32. Process according to one of claims 4 to 15, wherein a first crystallization of the paraxylene of step (d) is performed (figure 9 and 10) in a first crystallization zone at a temperature T1, a suspension of first paraxylene crystals in a first mother liquor is recovered, the first crystals are separated from the first mother liquor in a first separation zone (82), at least a portion of the first mother liquor is crystallized in a second crystallization zone (50) at a temperature T2 that is lower than T1, the second crystals are separated from the second mother liquor in a second separation zone (86), the second mother liquor is recovered that is recycled at least in part to the adsorption zone (8), the first crystals and the second crystals are recovered and they are washed in at least one separation and countercurrent washing zone (110) with the suitable washing solvent, a washing liquor (115) which is recycled at least in part to the first crystallization zone (70) is recovered on the one hand, paraxylene crystals are recovered on the other hand, they are melted completely in a melting zone (112) and a stream of melted paraxylene is collected.

33. Process according to claim 32, wherein a portion of the stream of melted paraxylene is drawn off and used as washing solvent.

34. Process according to claim 32, wherein the washing solvent is the desorption solvent, the stream of melted paraxylene is distilled, washing solvent is collected that is recycled at least in part in the washing zone, the washing liquor is distilled before recycling it to the first crystallization zone and, at least in part, the washing solvent is recycled in the washing zone.

35. Process according to one of claims 21 to 34, wherein a portion of the first mother liquor is recycled to the first crystallization zone and a portion of the second mother liquor to the second crystallization zone.

36. Process according to one of claims 5 to 35, wherein the separation zone where the washing of the crystals is performed comprises at least one countercurrent washing column.

37. Process according to one of claims 5 to 35, wherein the separation zone where the washing of the crystals is performed comprises at least one centrifuge or at least one rotary filter.

38. Process according to one of claims 5 to 37, wherein said desorption solvent is toluene and wherein a portion of the charge is made to circulate in a distillation column (40) to draw off toluene (32) from it, at least in part, and said toluene is introduced as an added portion of desorption solvent.

39. Process according to one of claims 4 to 38, wherein the desorption solvent is either toluene or paradiethylbenzene.

## Patentansprüche

1. Verfahren zum (Ab)trennen und Gewinnen des Paraxylols aus einer Charge, die Metaxylol enthalten kann, wobei das Verfahren in Kombination einen Vorgang der Adsorption und einen Vorgang der Kristallisation umfasst, **dadurch gekennzeichnet, dass** wenigstens ein Teil der Charge und/oder der aus der Kristallisation resultierenden Stammlösung einer Bleicherdebehandlung unterzogen wird, wobei diese Erde aus der gegebenenfalls aktivierte natürliche Silicoaluminate umfassenden Gruppe gewählt ist und die Temperatur zwischen 100 und 300°C, der Druck zwischen 3 und 100 bar und die stündliche Raumgeschwindigkeit zwischen 1 bis 8 beträgt.

2. Verfahren nach Anspruch 1, in Kombination über die Vorgänge der Adsorption und Kristallisation hinaus eine Stufe der ein Isomerat liefernden Isomerierung umfasst, **dadurch gekennzeichnet, dass** wenigstens ein Teil des Isomerats mit Bleicherde behandelt wird.

3. Verfahren nach Anspruch 2, **dadurch gekennzeichnet, dass** wenigstens ein Teil der Charge und wenigstens ein Teil des Isomerats je einer Bleicherdebehandlung unterzogen werden.

4. Verfahren zum Trennen und Gewinnen von Paraxylol, das in einer aromatische C₈-Kohlenwasserstoffe umfassenden Kohlenwasserstoffcharge enthalten ist, wobei man insbesondere:
- in wenigstens einer Adsorptionszone (8) diese wenigstens Metaxylol, Paraxylol mit wenigstens einem Adsorbens in Anwesenheit wenigstens eines geeigneten Desorptionslösungsmittels enthaltende Charge (1) unter Adsorptionsbedingungen derart kontaktiert, dass man eine erste Lösungsmittel, Metaxylol und gegebenenfalls Ethylbenzol und/oder Orthooxylol enthaltende erste Fraktion und eine zweite Lösungsmittel für Paraxylol in im wesentlichen angereichertem Zustand enthaltende zweite Fraktion erhält;
- die erste Fraktion destilliert (12), um ein Gemisch aus Metaxylol und gegebenenfalls Ethylbenzol und/oder Orthoxylol zu gewinnen;
- zum Teil dieses Gemisch unter geeigneten Bedingungen in wenigstens einer Isomerierungszone (21) isomeriert und ein Isomerat (2) gewinnt, das man wenigstens zum Teil zur Adsorptionszone rezykliert;
- diese zweite Fraktion destilliert (16), um im wesentlichen angereichertes Paraxylol zu gewinnen und man eine Kristallisation des im wesentlichen angereicherten Paraxylols unter Erhalt eines Teils einer Stammlösung vornimmt, die man wenigstens zum Teil zur Adsorptionsstufe rezykliert und andererseits Paraxylolkristalle erhält,
**dadurch gekennzeichnet, dass** man wenigstens einen Teil zirkulieren lässt, der gewählt ist wenigstens zum Teil aus der Charge (1), wenigstens zum Teil dem Isomerat (2) und wenigstens zum Teil der Stammlösung (3) (32) in wenigstens einer Zone (26) zur Behandlung mit Bleicherde oder äquivalentem Material, wobei die Bleicherde gewählt ist aus der Gruppe der natürlichen gegebenenfalls aktivierten Silicoaluminate, die Temperatur zwischen 100 bis 300°C, der Druck zwischen 3 und 100 bar und die stündliche Raumgeschwindigkeit bei 1 bis 8 liegt und man einen ersten Abstrom (27) gewinnt, den man wenigstens zum Teil in die Adsorptionszone (8) einführt, um diese ersten und zweiten Fraktionen zu erzeugen, die aus der Adsorptionszone abgezogen wurden.

5. Verfahren nach Anspruch 4, bei dem man die Paraxylolkristalle mit einem geeigneten Waschlösungsmittel in wenigstens einer Waschzone wäscht und Paraxylolkristalle bei sehr hohem Reinheitsgrad gewinnt.

6. Verfahren nach einem der Ansprüche 4 bis 5, bei dem man wenigstens einen Teil der Charge (1) und des Isomerats in wenigstens einer Zone der Bleicherdebehandlung zirkulieren lässt.

7. Verfahren nach einem der Ansprüche 4 bis 6, bei dem man wenigstens einen Teil der Charge, des Isomerats (2) und der Stammlösung in einer oder mehreren Bleicherdebehandlungszonen zirkulieren lässt.

8. Verfahren nach einem der Ansprüche 4 bis 7, **dadurch gekennzeichnet, dass** die Adsorptionszone im simulierten beweglichen Bett betrieben wird.

9. Verfahren nach einem der Ansprüche 4 bis 8, bei dem wenigstens ein Teil der Stammlösung (3) (33) in eine Destillationskolonne (23) hinter der Isomierungszone (21) eingeführt wird, man das Isomerat und die Stammlösung destilliert, eine Kopffraktion erhält, welche leichte Verbindungen und eine Fraktion erhält, welche ein Gemisch aus dieser Stammlösung und eines destillierten Isomerats enthält, das anschließend in die Zone (26) der Bleicherdebehandlung eingeführt wird und dass man einen zweiten Abstrom gewinnt.

10. Verfahren nach Anspruch 9, bei dem im übrigen eine schwere Verbindungen enthaltende Schwanzfraktion aus der Destillationskolonne (23) abgezogen wird.

11. Verfahren nach einem der Ansprüche 4 bis 10, bei dem ein Teil der Stammlösung (31) mit dem ersten oder zweiten Abstrom der Bleicherdebehandlungszone vermischt wird, und man einen dritten Abstrom gewinnt.

12. Verfahren nach einem der Ansprüche 4 bis 11, bei dem der erste, der zweite oder der dritte Abstrom der Bleicherdebehandlungszone in wenigstens einer Destillationskolonne (28) (sog, rerun-Kolonne) destilliert wird, die eine Schwanzfraktion (29) liefert, welche schwere Verbindungen und eine Kopffraktion (4) enthält, die in die Adsorptionszone (8) eingeführt wird.

13. Verfahren nach einem der Ansprüche 1 bis 12, bei dem die Bleicherdebehandlung bei einer Temperatur von 160 bis 230°C, einem Druck von 4 bis 20 bar und einer stündlichen Raumgeschwindigkeit von 1 bis 4 durchgeführt wird.

14. Verfahren nach einem der Ansprüche 4 bis 13, bei dem wenigstens ein Teil der Stammlösung gereinigt wird, bevor er in die Bleicherdebehandlungszone eingeführt wird.

15. Verfahren nach einem der Ansprüche 4 bis 14, bei dem das Desorptionslösungsmittel aus den Stufen (b) und (d) wenigstens zum Teil gereinigt wird, bevor es rezykliert wird und dass man die Reinigung des Lösungsmittels durch eine Zufuhr frischen Lösungsmittels entweder in die Charge oder in die Zone vor der Adsorptionszone kompensiert.

16. Verfahren nach einem der Ansprüche 4 bis 15, bei dem man eine Kristallisation (50) in einer einzigen Stufe des Paraxylols der Stufe (d) vornimmt, eine Suspension aus Kristallen (51) in der Stammlösung gewinnt und die Kristalle der Stammlösung in einer Trennzone (52) trennt, die mit dem Waschlösungsmittel erhaltenen Kristalle wäscht, eine Waschlösung oder Waschlauge gewinnt, wenigstens einen Teil der Stammlösung (53) und wenigstens einen Teil der Waschlösung rezykliert und man vollständig die Paraxylolkristalle (58) derart schmilzt, dass ein Flüssigkeitsstrom aus schmelzflüssigem Paraxylol erhalten wird.

17. Verfahren nach Anspruch 16, bei dem die Trenn- und Waschzone wenigstens eine Gegenstromwaschkolonne (80) ist, in die man als Waschflüssigkeit (59b) wenigstens einen Teil des flüssigen geschmolzenen Paraxylolstroms einführt und wobei die Stammlösung und die Waschlösung oder Waschflüssigkeit ein und die gleiche Lösung (53) sind, die man wenigstens zum Teil zur Adsorptionszone (8) und gegebenenfalls zum Teil zur Kristallisationszone (50) rezykliert.

18. Verfahren nach Anspruch 16, bei dem die Trenn- und Waschzone wenigstens eine Zentrifuge oder wenigstens ein Rotationsfilter ist, in das man als Waschlösungsmittel wenigstens einen Teil des flüssigen geschmolzenen Paraxylolstroms einführt und in dem die Stammlösung und die Waschlösung zur Adsorptionszone und gegebenenfalls zum Teil zur Kristallisationszone rezykliert werden.

19. Verfahren nach Anspruch 16, bei dem das Waschlösungsmittel das Desorptionslösungsmittel ist, man (60) den flüssigen Paraxylolstrom derart destilliert, dass in einer Kopffraktion Waschlösungsmittel (56) gewinnt, das man wenigstens zum Teil in die Waschzone (52) der Kristalle rezykliert und in eine Schwanzfraktion für das flüssige Paraxylol sehr hoher Reinheit rezykliert, sei es, dass man die Stammlösung (53) rezykliert, welche das Waschlösungsmittel enthält, und zwar gegen die Desoprtionszone (8) und gegebenenfalls zum Teil gegen die Kristallisationszone (50), nachdem man sie gegebenenfalls destilliert hat, wenn es sich bei der Waschzone um eine Gegenstromwaschkolonne handelt oder man rezykliert die Stammlösung (53) zur Adsorptionszone (8) und gegebenenfalls zum Teil gegen die Kristallisationszone (50) und die Waschlösung in eine Destillationszone gemäß Stufe (b), wenn die Waschzone eine Zentrifuge oder ein Rotationsfilter (52) ist.

20. Verfahren nach Anspruch 16, bei dem das Waschlösungsmittel ein Lösungsmittel außer dem Strom flüssigen Paraxylols und außer dem Desorptionslösungsmittel ist, man den flüssigen Strom aus schmelzflüssgem Paraxylol derart destilliert, dass man am Kopf Lösungsmittel gewinnt, das man wenigstens zum Teil in die Waschzone der Kristalle rezykliert und man flüssiges Paraxylol sehr hoher Reinheit schmilzt und indem man entweder die die Waschlösung enthaltende Stammlösung zur Adsorptionszone und gegebenenfalls zum Teil zur Kristallisationszone rezykliert, nachdem man sie destilliert hat, wobei das Lösungsmittel in der Waschzone rezykliert wird, wenn die Waschzone eine Gegenstromwaschzone ist oder man rezykliert die Stammlösung zur Adsorptionszone und gegebenenfalls zum Teil zur Kristallisationszone und die Waschlösung, nachdem sie destilliert wurde, zur Adsorptionszone und gegebenenfalls zum Teil zur Kristallisationszone, wobei das Lösungsmittel zur Waschzone dann rezykliert wird, wenn die Waschzone eine Zentrifuge oder ein rotierendes Filter ist.

21. Verfahren nach einem der Ansprüche 4 bis 15, bei dem man (Figuren 4 und 5) eine erste Kristallisation des Paraxylols der Stufe (d) in einer ersten Kristallisationszone (50) bei einer Temperatur T₁ durchführt, eine Suspension (51) der ersten Paraxylolkristalle in einer ersten Stammlösung gewinnt, die ersten Kristalle von der ersten Stammlösung in einer ersten Trennzone (52) trennt, wenigstens einen Teil der ersten Stammlösung zur Adsorptionszone (8) rezykliert, die ersten abgetrennten Kristalle (58) schmilzt und sie in einer zweiten Kristallisationszone (70) bei einer Temperatur T₂ oberhalb T₁ kristallisiert, eine Suspension (71) zweiter Paraxylolkristalle in einer zweiten Stammlösung gewinnt, die zweiten Kristalle von der zweiten Stammlösung in einer zweiten Trennzone (52) abtrennt, die zweiten Kristalle mit dem geeigneten Waschlösungsmittel in der zweiten Trennzone wäscht, eine Waschlösung gewinnt, wenigstens zum Teil die zweite Stammlösung (73) und wenigstens einen Teil der Waschlösung zur ersten Kristallisationszone und gegebenenfalls zum Teil zur zweiten Kristallisationszone rezykliert und vollständig (76) die zweiten Paraxylolkristalle derart schmilzt, dass ein Flüssigkeitsstrom (77) aus geschmolzenem Paraxylol erhalten wird.

22. Verfahren nach Anspruch 21, bei dem das Waschlösungsmittel ein Teil des flüssigen geschmolzenen Paraxylolstroms ist, und die Waschflüssigkeit und die zweite Stammlösung nur ein und die gleiche rezyklierte Lösung sind, die zum Teil zur ersten Kristallisationszone und gegebenenfalls zum Teil zur zweiten Kristallisationszone rezykliert werden.

23. Verfahren nach Anspruch 21, bei dem das Waschlösungsmittel das Desorptionslösungsmittel, beispielsweise Toluol, ist und bei dem man den Strom schmelzflüssigen Paraxylols destilliert (60), derart, dass man in einer Kopffraktion Waschlösungsmittel gewinnt, das man wenigstens zum Teil in die zweite Trennzone (72) rezykliert und in einer Schwanzfraktion flüssiges Paraxylol sehr hoher Reinheit und dann entweder gegebenenfalls die zweite Stammlösung, die die Waschlösung enthält, destilliert, bevor sie rezykliert wird, wenn die zweite Trennzone eine Gegenstromwaschkolonne ist oder man die Waschlösung destilliert, bevor man sie zur ersten Kristallisationseinheit und/oder gegen die zweite Kristallisationseinheit rezykliert, wenn die zweite Trennzone eine Zentrifuge oder ein Drehfilter ist.

24. Verfahren nach Anspruch 21, bei dem das Waschlösungsmittel ein Lösungsmittel außer dem Strom schmelzflüssigen Paraxylols und dem Desorptionslösungsmittel ist, man den Strom schmelzflüssigen Paraxylols, das aus der Suspension zweiter Kristalle stammt, destilliert (60), derart, dass man in einer Kopffraktion Waschlösungsmittel gewinnt, das man wenigstens zum Teil in die zweite Trennzone rezykliert und in einer Schwanzfraktion flüssiges Paraxylol sehr hoher Reinheit gewinnt und man entweder die Stammlösung destilliert, welche die Waschlösung umfasst, bevor sie zur ersten Kristallisationszone und gegebenenfalls im zum Teil gegen die zweite Kristallisationszone rezykliert wird, wenn die zweite Trennzone eine Gegenstromkolonne ist oder man die Stammlösung zur ersten Kristallisationszone und gegebenenfalls zum Teil zur zweiten Kristallisationszone rezykliert, man die Waschlösung destilliert, bevor sie zur ersten Kristallisationszone und gegebenenfalls zum Teil gegen die zweite Kristallisationszone rezykliert wird, wenn die zweite Trennzone eine Zentrifuge oder ein Drehfilter ist.

25. Verfahren nach einem der Ansprüche 4 bis 15, bei dem man (Figuren 6 und 7) eine erste Kristallisation des Paraxylols der Stufe (d) in einer ersten Kristallisationszone (70) bei einer Temperatur T1 vornimmt, eine Suspension (81) der ersten Kristalle des Paraxylols in einer ersten Stammlösung gewinnt, die ersten Kristalle der ersten Stammlösung in einer ersten Trennzone (82) abtrennt und gegebenenfalls die ersten Kristalle mit dem Waschlösungsmittel in der ersten Trennzone wäscht, wenigstens einen Teil der ersten Stammlösung in einer zweiten Kristallisationszone (50) bei einer Temperatur T2 unterhalb der Temperatur T1 kristallisiert, eine Suspension (85) zweiter Paraxylolkristalle in einer zweiten Stammlösung gewinnt, die zweiten Kristalle der zweiten Stammlösung in einer zweiten Trennzone (86) abtrennt und die zweiten Kristalle mit dem geeigneten Waschlösungsmittel in der Trennzone wäscht und eine zweite Stammlösung (87) gewinnt, die man wenigstens zum Teil zur Adsorptionszone (8) rezykliert und man dann die ersten und zweiten Paraxylolkristalle mischt, man sie vollständig schmilzt (89) und einen Strom (90) schmelzflüssigen Paraxylols gewinnt.

26. Verfahren nach Anspruch 25, bei dem wenigstens ein Teil des Stroms schmelzflüssigen Paraxylols entnommen und als Waschlösungsmittel in der ersten Trennzone (82) und der zweiten Trennzone (86) gewendet wird.

27. Verfahren nach Anspruch 25, bei dem das Waschlösungsmittel das Desorptionslösungsmittel, beispielsweise Toluol, ist und bei dem man den Strom schmelzflüssigen Paraxylols destilliert, eine Waschlösungsmittel enthaltende Kopffraktion gewinnt, die wenigstens zum Teil zur ersten und zweiten Trennzone rezykliert wird und eine Schwanzfraktion gewinnt, die flüssiges Paraxylol sehr hoher Reinheit enthält.

28. Verfahren nach einem der Ansprüche 5 bis 15, bei dem man wie folgt vorgeht: man führt eine erste (Figur 8) Kristallisation des Paraxylols der Stufe (d) und von gegebenenfalls schmelzflüssigen rezyklierten Kristallen durch, die aus einer zweiten nachstehend beschriebenen Kristallisation stammen, und zwar in einer ersten Kristallisationszone (70) bei einer Temperatur T1, man gewinnt eine Suspension (81) aus Paraxylolkristallen in einer ersten Stammlösung, man trennt die Kristalle der ersten Stammlösung in einer ersten Trennzone (82), man wäscht diese Kristalle in dieser Zone, man schmilzt sie (100) und man gewinnt einen Strom schmelzflüssigen Paraxylols, man kristallisiert wenigstens einen Teil der ersten Stammlösung (82) in einer zweiten Kristallisationszone (50) bei einer Temperatur T2, die unterhalb von T1 liegt, man gewinnt eine Suspension aus zweiten Kristallen von Paraxylol in einer zweiten Stammlösung, man trennt die zweiten Kristalle der zweiten Stammlösung in einer zweiten Trennzone (86), man gewinnt eine zweite Stammlösung (87), die man wenigstens zum Teil zur Adsorptionszone rezykliert und man schmilzt (103) gegebenenfalls die zweiten Kristalle und rezykliert die gegebenenfalls geschmolzenen Kristalle zur ersten Kristallisationszone, um sie mit dem Paraxylol der Stufe (d) bei der Temperatur T1 zu rekristallisieren.

29. Verfahren nach einem der Ansprüche 4 bis 15, bei dem man eine erste (Figur 8) Kristallisation des Paraxylols der Stufe (d) und von teilweise geschmolzenen rezyklierten Kristallen, die aus einer nachstehenden zweiten Kristallisation stammen, in einer ersten Kristallisationszone (70) bei einer Temperatur T1 vornimmt, man eine Suspension (81) aus Paraxylolkristallen in einer ersten Stammlösung gewinnt, die Kristalle von der ersten Stammlösung in einer zweiten Trennzone (82) trennt, diese Kristalle in dieser Zone wäscht, man sie schmilzt (100) und einen Strom schmelzflüssigen Paraxylols gewinnt, man wenigstens einen Teil der ersten Stammlösung (83) in einer zweiten Kristallisationszone (50) bei einer Temperatur T2, die unterhalb der Temperatur T1 liegt, kristallisiert, man eine Suspension von zweiten Paraxylolkristallen in einer zweiten Stammlösung gewinnt, man die zweiten Kristalle der zweiten Stammlösung in einer zweiten Trennzone (86) abtrennt, von dem man wenigstens einen Teil zur Adsorptionszone rezykliert, man (103) partiell die zweiten Kristalle schmilzt und die Kristalle in Suspenion zur ersten Kristallisationszone rezykliert, um sie mit dem Paraxylol der Stufe (d) bei der Temperatur T1 zu rekristallisieren.

30. Verfahren nach den Ansprüchen 28 und 29, bei dem wenigstens ein Teil des Stroms schmelzflüssigen Paraxylols, der aus der ersten Trennzone stammt, als Waschlösungsmittel verwendet wird.

31. Verfahren nach den Ansprüchen 28 und 29, bei dem das Waschlösungsmittel das Desorptionslösungsmittel ist und bei dem man den Strom schmelzflüssigen Paraxylols, der aus der ersten Kristallisationszone stammt, destilliert und in einer Kopffraktion Waschlösungsmittel gewinnt, das man wenigstens zum Teil in die erste Trennzone rezykliert und in einer Schwanzfraktion flüssiges Paraxylol sehr hoher Reinheit gewinnt.

32. Verfahren nach einem der Ansprüche 4 bis 15, bei dem man wie folgt vorgeht: man führt eine erste Kristallisation (Figur 9 und 10) des Paraxylols der Stufe (d) in einer ersten Kristallisationszone bei einer Temperatur T1 durch, man gewinnt eine Suspension aus ersten Paraxylolkristallen in einer ersten Stammlösung, man trennt die ersten Kristalle von der ersten Stammlösung in einer ersten Trennzone (82), man kristallisiert wenigstens einen Teil der ersten Stammlösung in einer zweiten Kristallisationszone (50) bei einer unterhalb der Temperatur T2 liegenden Temperatur T1, man trennt die zweiten Kristalle von der zweiten Stammlösung in einer zweiten Trennzone (86), man gewinnt die zweite Stammlösung, die man wenigstens zum Teil zur Adsorptionszone (8) rezykliert, gewinnt die ersten Kristalle und die zweiten Kristalle und mischt sie in wenigstens einer Trenn- und Gegenstromwaschzone (110) mit dem geeigneten Waschlösungsmittel, gewinnt einen Teil einer Waschlösung (115), die wenigstens zum Teil zur ersten Kristallisationszone (70) rezykliert wird und gewinnt andererseits Paraxylolkristalle, schmilzt sie vollständig in einer Schmelzzone (112) und sammelt einen Strom aus schmelzflüssigem Paraxylol.

33. Verfahren nach Anspruch 32, bei dem ein Teil des schmelzflüssigen Paraxylolstroms entnommen und als Waschlösungsmittel verwendet wird.

34. Verfahren nach Anspruch 32, bei dem das Waschlösungsmittel das Desorptionslösungsmittel ist, man destilliert den Strom schmelzflüssigen Paraxylols, sammelt Waschlösungsmittel, das man wenigstens zum Teil in die Waschzone rezykliert, destilliert die Waschflüssigkeit oder -lösung, bevor man sie zur ersten Kristallisationszone rezykliert und rezykliert wenigstens zum Teil das Waschlösungsmittel in die Waschzone.

35. Verfahren nach einem der Ansprüche 21 bis 34, bei dem ein Teil der ersten Stammlösung zur ersten Kristallisationszone und ein Teil der zweiten Stammlösung zur zweiten Kristallisationszone rezykliert wird.

36. Verfahren nach einem der Ansprüche 5 bis 35, bei dem die Trennzone, wo das Waschen der Kristalle stattfindet, wenigstens eine Gegenstromwaschkolonne umfasst.

37. Verfahren nach einem der Ansprüche 5 bis 35, bei dem die Trennzone, wo das Waschen der Kristalle stattfindet, wenigstens eine Zentrifuge oder wenigstens ein Rotationsfilter umfasst.

38. Verfahren nach einem der Ansprüche 5 bis 37, bei dem dieses Desorptionslösungsmittel Toluol ist und bei dem man einen Teil der Charge in einer Destillationskolonne (40) zirkulieren lässt, um hieraus wenigstens einen Teil des Toluols (32) abzuziehen und man führt dieses Toluol als Desorptionslösungsmittelzusatz ein.

39. Verfahren nach einem der Ansprüche 4 bis 38, **dadurch gekennzeichnet, dass** dieses Desorptionslösungsmittel entweder Toluol oder Paradiethylbenzol ist.
